# FASCICULE DE BREVET EUROPEEN

(11) **EP 4 003 587 B1**
(45) Date de publication et mention de la délivrance du brevet: **12.07.2023**
(21) Numéro de dépôt: 20739701.9
(22) Date de dépôt: 16.07.2020
(51) Int. Cl.: B01J 21/04, B01J 23/00, B01J 23/755, B01J 35/00, B01J 35/10, B01J 37/02, B01J 37/08, C07C 5/09, C07C 5/11, C10G 45/36, C10G 45/48

(54) **CATALYSEUR COMPRENANT UNE PHASE ACTIVE DE NICKEL SOUS FORME DE PETITES PARTICULES REPARTIE EN CROUTE ET UN ALLIAGE NICKEL CUIVRE**
KATALYSATOR MIT EINER AKTIVEN NICKELPHASE IN FORM VON IN EINER HÜLLE VERTEILTEN KLEINEN PARTIKELN UND EINER NICKEL-KUPFER-LEGIERUNG
CATALYST COMPRISING AN ACTIVE NICKEL PHASE IN THE FORM OF SMALL PARTICLES DISTRIBUTED IN A SHELL AND A NICKEL-COPPER ALLOY

(30) Priorité: 31.07.2019 FR 1908724
(43) Date de publication de la demande: 01.06.2022
(73) Titulaire: IFP Energies nouvelles, 92852 Rueil-Malmaison Cedex (FR)
(72) Inventeur: DUBREUIL, Anne-Claire, 92852 Rueil-Malmaison Cedex (FR); BOUALLEG, Malika, 92852 Rueil-Malmaison Cedex (FR)
(74) Mandataire: IFP Energies nouvelles
(86) Numéro de dépôt international: PCT/EP2020/070078
(87) Numéro de publication internationale: WO 2021/018600

(56) Documents cités:
- FR-A1- 2 927 267
- FR-A1- 3 064 500
- FR-A1- 3 076 746
- US-A- 5 948 942
- US-A1- 2012 065 442
- MIN KANG ET AL: "[gamma]-Alumina supported Cu-Ni bimetallic catalysts: Characterization and selective hydrogenation of 1,3-butadiene", CANADIAN JOURNAL OF CHEMICAL ENGINEERING, vol. 80, no. 1, 1 février 2002 (2002-02-01), pages 63-70, XP055672478, US ISSN: 0008-4034, DOI: 10.1002/cjce.5450800107

## Description

### Domaine technique

La présente invention concerne un catalyseur métallique supporté à base de nickel et de cuivre destiné particulièrement à l'hydrogénation des hydrocarbures insaturés, et plus particulièrement, d'hydrogénation sélective de composés polyinsaturés ou d'hydrogénation des aromatiques.

### Etat de la technique

Les composés organiques mono-insaturés tels que par exemple l'éthylène et le propylène, sont à la source de la fabrication de polymères, de matières plastiques et d'autres produits chimiques à valeur ajoutée. Ces composés sont obtenus à partir du gaz naturel, du naphta ou du gazole qui ont été traités par des procédés de vapocraquage ou de craquage catalytique. Ces procédés sont opérés à haute température et produisent, en plus des composés mono-insaturés recherchés, des composés organiques polyinsaturés tels que l'acétylène, le propadiène et le méthylacétylène (ou propyne), le 1-2-butadiène et le 1-3-butadiène, le vinylacétylène et l'éthylacétylène, et d'autres composés polyinsaturés dont le point d'ébullition correspond à la fraction essence C5+ (essences contenant des composés hydrocarbonés ayant 5 atomes de carbone ou plus), en particulier des composés styréniques ou indéniques. Ces composés polyinsaturés sont très réactifs et conduisent à des réactions parasites dans les unités de polymérisation. Il est donc nécessaire de les éliminer avant de valoriser ces coupes.

L'hydrogénation sélective est le principal traitement développé pour éliminer spécifiquement les composés polyinsaturés indésirables de ces charges d'hydrocarbures. Elle permet la conversion des composés polyinsaturés vers les alcènes ou aromatiques correspondants en évitant leur saturation totale et donc la formation des alcanes ou naphtènes correspondants.

Les catalyseurs d'hydrogénation sélective sont généralement à base de métaux du groupe VIII du tableau périodique, de préférence le palladium ou le nickel. Le métal se présente sous la forme de particules métalliques déposées sur un support. La teneur en métal, la taille des particules de métal et la répartition de la phase active dans le support font partie des critères qui ont une importance sur l'activité et la sélectivité des catalyseurs.

La répartition macroscopique des particules métalliques dans le support constitue un critère important, principalement dans le cadre de réactions rapides et consécutives telles que les hydrogénations sélectives. Il est généralement souhaitable que ces éléments se situent dans une croûte à la périphérie du support afin d'éviter les problèmes de transfert de matière intragranulaire pouvant conduire à des défauts d'activité et une perte de sélectivité. De tels catalyseurs sont aussi appelé catalyseurs "eggshell" selon la terminologie anglo-saxonne. De tels catalyseurs sont largement connus dans le cas des catalyseurs d'hydrogénation sélective à base de palladium. En effet, grâce à la faible teneur en palladium (généralement inférieure à 1 % en poids (1 % pds) de palladium par rapport au catalyseur) et des procédés de préparation adaptés, une croûte fine de palladium à la périphérie des grains de support peut être obtenue (FR2922784, US2010/217052).

Il est souvent proposé de substituer le palladium par le nickel, métal moins actif que le palladium qu'il est donc nécessaire de disposer en plus grande quantité dans le catalyseur. Ainsi, les catalyseurs à base de nickel ont généralement une teneur en métal entre 5 et 50 % pds de nickel par rapport au catalyseur. Dans ces catalyseurs, le nickel est généralement réparti de façon homogène au sein du support. Une des voies d'amélioration possible de ces catalyseurs en termes d'activité et de sélectivité est de contrôler la répartition du nickel au sein du support en déposant le nickel de façon plus concentrée sur une croûte, à la périphérie du support. De tels catalyseurs sont connus de l'état de l'art.

Le document US 4 519 951 décrit un catalyseur de type « eggshell » avec du nickel sur un support poreux ayant un volume poreux des pores dont la taille est inférieure à 11,7 nm d'au moins 0,2 ml/g et un volume poreux des pores dont la taille est supérieure à 11,7 nm d'au moins 0,1 ml/g. Plus de 50 % du nickel se trouve dans une croûte dont l'épaisseur est égale à 0,15 fois le rayon du support. Ce catalyseur est utilisé pour l'hydrogénation de matières grasses.

Le document CN101890351 décrit un catalyseur supporté de nickel dans lequel plus de 90 % du nickel se trouve dans une croûte de 700 µm d'épaisseur. Le catalyseur est préparé en utilisant une solution ammoniacale pour dissoudre le sel de nickel. Ces catalyseurs sont utilisés dans une application d'hydrogénation sélective.

Le document US2012/0065442 décrit un catalyseur supporté de nickel dans lequel la distribution de la taille des cristallites de nickel est bimodale avec 30 à 70% des cristallites de nickel ayant une taille moyenne (diamètre) de 1,0 à 2,5 nm, les cristallites de nickel restants ayant une taille moyenne (diamètre) de 3,0 à 4,5 nm. Le nickel est reparti à la fois sur une croûte d'une épaisseur de 3 à 15 % du diamètre et à coeur, le ratio de concentration en nickel entre la croûte et le coeur étant compris entre 3,0 : 1 et 1,3 : 1. Au moins 75 % du volume poreux de trouve dans des pores ayant une taille de plus de 5,0 nm.

La demande FR3064500 divulgue un catalyseur comprenant au moins un métal du groupe VIII et un support comprenant au moins un oxyde réfractaire sélectionné dans le groupe constitué par la silice, l'alumine et la silice-alumine, ledit catalyseur étant obtenu par un procédé de préparation comprenant au moins les étapes suivantes :
a) on met en contact ledit support poreux avec au moins une solution contenant au moins un compose organique comprenant au moins une fonction acide carboxylique ;
b) on met en contact ledit support poreux avec au moins une solution contenant au moins un précurseur de métal du groupe VIII ;
   les étapes a) et b) étant réalisées séparément, dans un ordre indiffèrent, ou simultanément;
c) on sèche le support imprégné a une température comprise entre 15°Cet inférieure ou égale à 250°C ;
d) on calcine le support sèche issu de l'étape c) a une température supérieure à 250°C mais inférieure à 900°C.

La demande FR2927267 divulgue un procédé de préparation d'un catalyseur d'hydrogénation sélective comprenant au moins les étapes suivantes :
a) on approvisionne un gel d'alumine de type hydrargilite ;
b) on met en forme le gel de l'étape a) par calcination flash, broyage, lavage et granulation ;
c) on soumet le gel d'alumine mis en forme obtenu à l'issue de l'étape b) à un traitement thermique comprenant au moins une étape de traitement hydrothermal dans un autoclave en présence d'une solution acide à une température de 200°C puis on réalise une étape de calcination à une température de 950°C pour obtenir un support d'alumine ;
d) on imprègne le support d'alumine obtenu à l'issue de l'étape c) avec une solution aqueuse de nitrate de nickel pour obtenir un précurseur de catalyseur ;
e) on sèche le précurseur de catalyseur obtenu à l'issue de l'étape d) à une température de 120°C ;
e') on calcine le précurseur de catalyseur obtenu à l'issue de l'étape e) à une température de 450°C pour obtenir un catalyseur ;
f) on met en contact le catalyseur obtenu à l'issue de l'étape e') avec au moins une solution contenant de l'acide formique ;
g) on réalise un traitement thermal du précurseur de catalyseur obtenu à l'issue de l'étape f) à une température de 150°C.

La demande FR3076746 divulgue un procédé de préparation d'un catalyseur comprenant une matrice oxyde et une phase active comprenant du nickel comprenant les étapes suivantes :
- on prépare un oxyde poreux aluminique calciné ;
- on malaxe l'oxyde poreux aluminique calciné obtenu avec au moins une solution comprenant au moins un précurseur de nickel pour obtenir une pâte, à une concentration en nickel voulue pour obtenir sur le catalyseur séché ou calciné une teneur comprise entre 10 et 35 % poids de nickel par rapport au poids total du catalyseur ;
- on met en forme la pâte obtenue ;
- on sèche la pâte mise en forme obtenue à une température inférieure à 250°C pour obtenir un précurseur de catalyseur séché ;
- on imprègne le précurseur de catalyseur séché obtenu avec au moins une solution comprenant au moins un précurseur de nickel, pour obtenir un précurseur de catalyseur imprégné ;
- on sèche le précurseur de catalyseur imprégné obtenu à une température inférieure à 250°C pour obtenir un catalyseur séché.

Le brevet US5948942 enseigne que la présence de cuivre dans un catalyseur de nickel peut permettre d'améliorer les performances en hydrogénation sélective des diènes en modifiant la réductibilité du nickel.

Dans la publication « Gamma-alumina supported Cu-Ni bimetallic catalysts : Characterization and sélective hydrogénation of 1 ,3-butadiène », Canadian Journal of Chemical Engineering, vol.80, no. 1, 1 février 2002 (2002-02-01), pages 63-70, Min Kang et al enseigne que l'introduction de cuivre dans un catalyseur de nickel sur support d'alumine conduit à un effet d'alliage à la formation d'une phase mixte cuivre-nickel et à la diminution de la température de réduction du catalyseur.

### Objets de l'invention

De manière surprenante, la Demanderesse a découvert qu'en appliquant un traitement hydrothermal spécifique après l'ajout d'un additif organique particulier sur un catalyseur à base de nickel et de cuivre (et dans lequel on forme un alliage à base de nickel et de cuivre sur le support) comprenant un support d'alumine obtenu selon une méthode bien particulière, on obtient un catalyseur dans lequel au moins une partie du nickel est répartie sur une croûte à la périphérie du support, l'autre partie du nickel étant répartie au coeur du catalyseur. Sans vouloir être lié par une quelconque théorie, le traitement hydrothermal réalisé après l'étape de mise en contact d'un additif organique spécifique sur le catalyseur à base de nickel et de cuivre sur un support d'alumine particulier, ayant subi un traitement hydrothermal en présence d'une solution acide, semble faire migrer au moins en partie le nickel de l'intérieur du support à la périphérie du support formant ainsi une croûte de nickel. De plus, il a été constaté par la Demanderesse que lors de la préparation du catalyseur, la réalisation d'une étape de mise en contact du support avec une solution contenant simultanément un précurseur métallique à base de cuivre et un précurseur métallique à base de nickel suivie d'une étape de séchage et de réduction en présence d'un gaz réducteur à basse température (comprise entre 150°C et 250°C) permet d'obtenir un alliage de nickel-cuivre (sous forme réduite) qui permet de manière inattendue d'améliorer fortement la réductibilité de la phase active de nickel sur le support. Par ailleurs, la présence de cuivre dans le catalyseur permet de maintenir une bonne activité et une durée de vie plus longue du catalyseur lorsque ce dernier est mis en contact avec une charge hydrocarbonée comprenant du soufre. En effet, par rapport au nickel, le cuivre présent dans le catalyseur capte plus facilement les composés soufrés compris dans la charge, ce qui limite l'empoisonnement irréversible des sites actifs.

La présente invention concerne ainsi un nouveau type de catalyseur qui, de par son procédé de préparation spécifique, permet d'obtenir un catalyseur comprenant des performances au moins aussi bonnes, voir meilleures, en terme d'activité et de sélectivité dans le cadre des réactions d'hydrogénation sélective de composés polyinsaturés ou d'hydrogénation des aromatiques polyinsaturés, tout en utilisant une quantité de phase de nickel inférieure que celle utilisée typiquement dans l'état de la technique, ce qui est due à une meilleure répartition de la phase active de nickel dans le support, rendant cette dernière plus accessible aux réactifs ainsi qu'à une taille de particule de nickel inférieure à 7 nm, conférant une activité intrinsèque du nickel encore plus importante. La présence d'un alliage NiCu permet également de réaliser une étape de réduction des éléments métalliques en présence d'un gaz réducteur à des températures plus basses et des temps de réaction plus court que ceux couramment utilisés dans l'art antérieur. Avantageusement, le recours à des conditions opératoires moins sévères que dans l'art antérieur permet de réaliser directement l'étape de réduction au sein du réacteur dans lequel on souhaite réaliser l'hydrogénation sélective de coupes polyinsaturés.

Un premier objet selon l'invention concerne un catalyseur comprenant du nickel et du cuivre, à raison de 1 et 50 % en poids en élément nickel par rapport au poids total du catalyseur, et d'un second élément métallique de cuivre, à raison de 0,5 à 15 % en poids en élément cuivre par rapport au poids total du catalyseur, et un support d'alumine, ledit catalyseur étant caractérisé en ce que :
- le nickel est réparti à la fois sur une croûte en périphérie du support, et à coeur du support, l'épaisseur de ladite croûte étant comprise entre 2% et 15% du diamètre du catalyseur ;
- le ratio de densité en nickel entre la croûte et le coeur est supérieur strictement à 3 ;
- ladite croûte comprend plus de 25% en poids élément nickel par rapport au poids total de nickel contenu dans le catalyseur ;
- le ratio molaire entre le nickel et le cuivre est compris entre 0,5 et 5 ;
- au moins une partie du nickel et du cuivre se présente sous la forme d'un alliage de nickel-cuivre ;
- la teneur en nickel comprise dans l'alliage nickel-cuivre est comprise entre 0,5 et 15% en poids en élément nickel par rapport au poids total du catalyseur,
- la taille des particules de nickel dans le catalyseur est inférieure à 7 nm. Avantageusement, le ratio de densité en nickel entre la croûte et le coeur est compris entre 3,8 et 15.

Avantageusement, ladite croûte comprend plus de 40% en poids élément nickel par rapport au poids total de nickel contenu dans le catalyseur.

Avantageusement, l'intervalle de transition entre le coeur et la croûte du catalyseur est compris entre 0,05% et 3% du diamètre du catalyseur.

Avantageusement, la teneur en soufre du support d'alumine est comprise entre 0,001% et 2% poids par rapport au poids total du support d'alumine, et la teneur en sodium dudit support d'alumine est comprise entre 0,001% et 2% poids par rapport au poids total dudit gel d'alumine.

Avantageusement, l'épaisseur de ladite croûte est comprise entre 2,5% et 12% du diamètre du catalyseur.

Avantageusement, le ratio de densité en nickel entre la croûte et le coeur est supérieur à 3,5.

Un autre objet selon l'invention concerne un procédé de préparation d'un catalyseur selon l'invention, ledit procédé étant caractérisé en ce que :
a) on approvisionne un gel ;
b) on met en forme le gel d'alumine de l'étape a) ;
c) on soumet le gel d'alumine mis en forme obtenu à l'issue de l'étape b) à un traitement thermique comprenant au moins une étape de traitement hydrothermal dans un autoclave en présence d'une solution acide, à une température comprise entre 100 et 800°C, et au moins une étape de calcination, à une température comprise entre 400 et 1500°C, réalisée après l'étape de traitement hydrothermal, pour obtenir un support d'alumine ;
d) on réalise l'enchaînement des sous-étapes suivantes :
   d1) on met en contact le support d'alumine avec au moins un précurseur de nickel pour obtenir un précurseur de catalyseur,
   d2) on sèche le précurseur de catalyseur obtenu à l'issue de l'étape d1) à une température inférieure à 250°C ;
   d3) on met en contact le précurseur de catalyseur séché obtenu à l'issue de l'étape d2) avec au moins une solution contenant au moins un additif organique choisi parmi les aldéhydes renfermant 1 à 14 atomes de carbone par molécule, les cétones ou polycétones renfermant 3 à 18 atomes de carbone par molécule, les éthers et les esters renfermant 2 à 14 atomes de carbone par molécule, les alcools ou polyalcools renfermant 1 à 14 atomes de carbone par molécule et les acides carboxyliques ou polyacides carboxyliques renfermant 1 à 14 atomes de carbone par molécule, le ratio molaire entre l'additif organique et le nickel étant supérieur à 0,05 mol/mol ;
   d4) on réalise un traitement hydrothermal du précurseur de catalyseur obtenu à l'issue de l'étape d3) à une température comprise entre 100 et 200°C pendant une durée comprise entre 30 minutes et 5 heures sous flux gazeux comprenant entre 5 et 650 grammes d'eau par kg de gaz sec ;
e) on réalise l'enchaînement des sous-étapes suivantes :
   e1) on met en contact le support d'alumine avec au moins une solution contenant au moins un précurseur de cuivre et un précurseur de nickel à une concentration en nickel voulue pour obtenir sur le catalyseur final une teneur comprise entre 0,5 et 15 % poids en élément nickel par rapport au poids total du catalyseur final ;
   e2) on réalise au moins une étape de séchage du précurseur de catalyseur obtenu à l'issue de l'étape e1) à une température inférieure à 250°C ;
      les étapes d) et e) étant réalisées séparément dans un ordre indifférent,
f) on met en contact le support d'alumine avec au moins une solution contenant au moins un composé organique comprenant au moins une fonction acide carboxylique, ou au moins une fonction alcool, ou au moins une fonction ester, ou au moins une fonction amide, ou au moins une fonction amine,
   l'étape f) étant réalisée, soit en même temps que la sous-étape d1) de l'étape d), soit avant soit après l'étape d), mais avant l'étape g), étant entendu que lorsque l'étape f) est réalisée avant ou après l'étape d), alors ladite étape f) inclut un séchage du précurseur de catalyseur à une température inférieure à 250°C après la mise en contact du support avec ladite solution comprenant au moins un composé organique;
g) on réduit le précurseur de catalyseur issu des étapes a) à f) par mise en contact dudit précurseur de catalyseur avec un gaz réducteur à une température supérieure ou égale à 150°C et inférieure à 250°C.

Avantageusement, le rapport molaire entre ledit composé organique introduit à l'étape f) et l'élément nickel également introduit à l'étape d1) est compris entre 0,01 et 5,0 mol/mol.

Avantageusement, les étapes d1) et f) sont réalisées simultanément.

Avantageusement, le composé organique de l'étape f) est choisi parmi l'acide oxalique, l'acide malonique, l'acide glycolique, l'acide acide lactique, l'acide tartronique, l'acide citrique, l'acide tartrique, l'acide pyruvique, l'acide lévulinique, l'éthylène glycol, le propane-1,3-diol, le butane-1 ,4-diol, le glycérol, le xylitol, le mannitol, le sorbitol, le glycol, le glucose, le carbonate de diméthyle, le carbonate de diéthyle la formamide, la N-méthylformamide, l'acétamide, la N-méthylacétamide, la N,N-diméthylméthanamide, la 2-pyrrolidone, la γ-lactame, la lactamide, l'urée, l'alanine, l'arginine, la lysine, la proline, la sérine, l'EDTA. Avantageusement, le précurseur de cuivre est choisi parmi l'acétate de cuivre, l'acétylacétonate de cuivre, le nitrate de cuivre, le sulfate de cuivre, le chlorure de cuivre, le bromure de cuivre, l'iodure de cuivre ou le fluorure de cuivre.

Avantageusement, à l'étape d3), l'additif organique est choisi parmi l'acide formique, le formaldéhyde, l'acide acétique, l'acide citrique, l'acide oxalique, l'acide glycolique, l'acide malonique, l'éthanol, le méthanol, le formiate d'éthyle, le formiate de méthyle, le paraldéhyde, l'acétaldéhyde, l'acide gamma-valérolactone, le glucose, le sorbitol et le trioxane.

Avantageusement, le ratio molaire entre l'additif organique introduit à l'étape d2) et le nickel est compris entre 0,1 et 5 mol/mol.

Avantageusement, le composé organique de l'étape f) est différent de l'additif organique de l'étape d2).

Un autre objet selon l'invention concerne un procédé d'hydrogénation sélective de composés polyinsaturés contenant au moins 2 atomes de carbone par molécule contenus dans une charge d'hydrocarbures ayant un point d'ébullition final inférieur ou égal à 300°C, lequel procédé étant réalisé à une température comprise entre 0 et 300°C, à une pression comprise entre 0,1 et 10 MPa, à un ratio molaire hydrogène/(composés polyinsaturés à hydrogéner) compris entre 0,1 et 10 et à une vitesse volumique horaire comprise entre 0,1 et 200 h⁻¹ lorsque le procédé est réalisé en phase liquide, ou à un ratio molaire hydrogène/(composés polyinsaturés à hydrogéner) compris entre 0,5 et 1000 et à une vitesse volumique horaire entre 100 et 40000 h⁻¹ lorsque le procédé est réalisé en phase gazeuse, en présence d'un catalyseur selon l'invention.

Un autre objet selon l'invention concerne un procédé d'hydrogénation d'au moins un composé aromatique ou polyaromatique contenu dans une charge d'hydrocarbures ayant un point d'ébullition final inférieur ou égal à 650°C, ledit procédé étant réalisé en phase gazeuse ou en phase liquide, à une température comprise entre 30 et 350°C, à une pression comprise entre 0,1 et 20 MPa, à un ratio molaire hydrogène/(composés aromatiques à hydrogéner) entre 0,1 et 10 et à une vitesse volumique horaire V.V.H. comprise entre 0,05 et 50 h⁻¹, en présence d'un catalyseur selon l'invention.

### Description de la figure

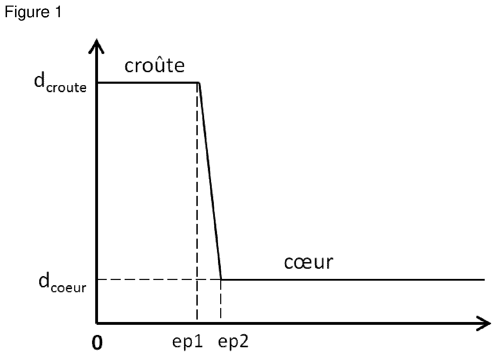

La figure 1 est un schéma représentant la répartition du nickel dans le catalyseur. L'axe des abscisses correspond à l'épaisseur du catalyseur, mesurée depuis le bord du catalyseur (en µm). L'axe des ordonnées correspond à la densité en nickel (en gramme de Ni / mm³). Le nickel est réparti à la fois sur une croûte en périphérie du support, d'épaisseur ep1, et à coeur du support. La densité en nickel sur la croûte d_{croute} est supérieure à la densité en nickel au coeur du support d_{coeur}. L'intervalle de transition entre le coeur et la croûte du catalyseur a une épaisseur notée ep2-ep1.

### Description détaillée de l'invention

### 1. Définitions

Dans la suite, les groupes d'éléments chimiques sont donnés selon la classification CAS (CRC Handbook of Chemistry and Physics, éditeur CRC press, rédacteur en chef D.R. Lide, 81ème édition, 2000-2001). Par exemple, le groupe VIII selon la classification CAS correspond aux métaux des colonnes 8, 9 et 10 selon la nouvelle classification IUPAC.

Dans la présente description, on entend, selon la convention IUPAC, par micropores les pores dont le diamètre est inférieur à 2 nm, c'est à dire 0,002 µm; par mésopores les pores dont le diamètre est supérieur ou égal à 2 nm, c'est à dire 0,002 µm et inférieur ou égal à 50 nm, c'est à dire 0,05 µm et par macropores les pores dont le diamètre est supérieur à 50 nm, c'est à dire 0,05 µm.

Afin d'analyser la répartition de la phase métallique sur le support, on mesure une épaisseur de croûte par microsonde de Castaing (ou microanalyse par microsonde électronique). L'appareil utilisé est un CAMECA XS100, équipé de quatre cristaux monochromateurs permettant l'analyse simultanée de quatre éléments. La technique d'analyse par microsonde de Castaing consiste en la détection de rayonnement X émis par un solide après excitation de ses éléments par un faisceau d'électrons de hautes énergies. Pour les besoins de cette caractérisation, les grains de catalyseur sont enrobés dans des plots de résine époxy. Ces plots sont polis jusqu'à atteindre la coupe au diamètre des billes ou extrudés puis métallisés par dépôt de carbone en évaporateur métallique. La sonde électronique est balayée le long du diamètre de cinq billes ou extrudés pour obtenir le profil de répartition moyen des éléments constitutifs des solides. Cette méthode, bien connue de l'Homme du métier, est définie dans la publication de L. Sorbier et al. "Measurement of palladium crust thickness on catalyst by EPMA" Materials Science and Engineering 32 (2012). Elle permet d'établir le profil de répartition d'un élément donné, ici le Nickel, au sein du grain. Par ailleurs, la concentration en Ni est définie pour chaque mesure et donc pour chaque pas d'analyse. La densité de Ni au sein du grain est donc définie comme la concentration de Ni par mm³.

Le volume poreux total est mesuré par porosimétrie au mercure selon la norme ASTM D4284-92 avec un angle de mouillage de 140°, par exemple au moyen d'un appareil modèle Autopore III^{™} de la marque Microméritics^{™}.

La surface spécifique BET est mesurée par physisorption à l'azote selon la norme ASTM D3663-03, méthode décrite dans l'ouvrage Rouquerol F.; Rouquerol J.; Singh K. « Adsorption by Powders & Porous Solids: Principle, methodology and applications », Academic Press, 1999.

On définit également le diamètre médian mésoporeux comme étant le diamètre tel que tous les pores, parmi l'ensemble des pores constituant le volume mésoporeux, de taille inférieure à ce diamètre constituent 50% du volume mésoporeux total déterminé par intrusion au porosimètre à mercure.

On entend par « taille des particules de nickel » le diamètre des cristallites de nickel sous forme oxyde. Le diamètre des cristallites de nickel sous forme oxyde est déterminé par diffraction des rayons X, à partir de la largeur de la raie de diffraction située à l'angle 2thêta=43° (c'est-à-dire selon la direction cristallographique [200]) à l'aide de la relation de Scherrer. Cette méthode, utilisée en diffraction des rayons X sur des poudres ou échantillons polycristallins qui relie la largeur à mi-hauteur des pics de diffraction à la taille des particules, est décrite en détail dans la référence : Appl. Cryst. (1978), 11, 102-113 « Scherrer after sixty years: A survey and some new results in the détermination of crystallite size», J. I. Langford and A. J. C. Wilson.

La teneur en nickel et en cuivre est mesurée par fluorescence X.

### 2. Catalyseur

L'invention porte sur un catalyseur à base de nickel et de cuivre, à raison de 1 et 50 % en poids en élément nickel par rapport au poids total du catalyseur, et de 0,5 à 15 % en poids en élément cuivre par rapport au poids total du catalyseur, et un support d'alumine, ledit catalyseur étant caractérisé en ce que :
- le nickel est réparti à la fois sur une croûte en périphérie du support, et à coeur du support, l'épaisseur de croûte (appelée aussi ep1) étant comprise entre 2% et 15% du diamètre du catalyseur, de préférence entre 2,5% et 12% du diamètre du catalyseur, de façon encore plus préférée entre 3% et 10 % du diamètre du catalyseur et de façon encore plus préférée entre 3% et 7,5% du diamètre du catalyseur ;
- le ratio de densité en nickel entre la croûte et le coeur (appelé aussi ici d_{croute}/d_{coeur}) est supérieur strictement à 3, de préférence supérieur à 3,5 et de préférence compris entre 3,8 et 15 ;
- ladite croûte comprend plus de 25% en poids en élément nickel par rapport au poids total de nickel contenu dans le catalyseur, de préférence plus de 40% en poids, plus préférentiellement entre 45% et 90% en poids, et encore plus préférentiellement entre 60 et 90% en poids ;
- le ratio molaire entre le nickel et le cuivre est compris entre 0,5 et 5 mol/mol, de préférence compris entre 0,7 et 4,5 mol/mol, plus préférentiellement entre 0,9 et 4 mol/mol ;
- au moins une partie du nickel et du cuivre se présente sous la forme d'un alliage de nickel-cuivre, répondant avantageusement à la formule NiₓCu_{y} avec x compris entre 0,1 et 0,9 et y compris entre 0,1 et 0,9 ;
- la teneur en nickel comprise dans l'alliage cuivre-nickel est comprise entre 0,5 et 15% en poids en élément nickel par rapport au poids total du catalyseur, de préférence entre 1 et 12% en poids, et plus préférentiellement entre 1 et 10% en poids ;
- la taille des particules de nickel, mesurée sous forme oxyde, dans le catalyseur est inférieure à 7 nm, de préférence inférieure à 5 nm, plus préférentiellement inférieure à 4 nm, et encore plus préférentiellement inférieure à 3 nm.

Avantageusement, l'intervalle de transition entre le coeur et la croûte du catalyseur (appelé aussi ici intervalle de transition coeur/croûte, ou ep2-ep1 d'après les notations de la figure 1), lié à la variation de la densité de nickel mesurée sur l'épaisseur du catalyseur depuis le bord du catalyseur jusqu'au centre du catalyseur, est très abrupte. De préférence, l'intervalle de transition coeur/ croûte est compris entre 0,05 % et 3 % du diamètre du catalyseur, de préférence entre 0,5 % et 2,5 % du diamètre du catalyseur.

La teneur en nickel dans ledit catalyseur selon l'invention est avantageusement comprise entre 1 et 50 % poids par rapport au poids total du catalyseur, plus préférentiellement entre 2 et 40 % poids et encore plus préférentiellement entre 3 et 35 % poids et encore plus préférentiellement 5 et 25% poids par rapport au poids total du catalyseur

La teneur en cuivre est comprise entre 0,5 et 15 % en poids en élément cuivre par rapport au poids total du catalyseur, de préférence comprise entre 0,5 et 12 % poids, de manière préférée comprise entre 0,75 et 10 % poids, et encore plus préférentiellement entre 1 et 9 % en poids.

Le catalyseur selon l'invention peut être qualifié comme catalyseur « semi egg-shell » dans lequel la concentration du nickel est plus élevée en périphérie du support que dans le coeur du support, ladite concentration du nickel dans le coeur du support étant non nulle.

La surface spécifique du catalyseur est généralement comprise entre 10 m²/g et 200 m²/g, de préférence entre 25 m²/g et 110 m²/g, de façon plus préférée entre 40 m²/g et 100 m²/g.

Le volume poreux total du catalyseur est généralement compris entre 0,1 et 1 ml/g, de préférence compris entre 0,2 ml/g et 0,8 ml/g, et de manière particulièrement préférée compris entre 0,3 ml/g et 0,7 ml/g.

La phase active du catalyseur ne comprend pas de métal du groupe VIB. Elle ne comprend notamment pas de molybdène ou de tungstène.

Ledit catalyseur (et le support utilisé pour la préparation du catalyseur) est sous forme de grains ayant avantageusement un diamètre compris entre 0,5 et 10 mm. Les grains peuvent avoir toutes les formes connues de l'Homme du métier, par exemple la forme de billes (ayant de préférence un diamètre compris entre 1 et 8 mm), d'extrudés, de tablettes, de cylindres creux. De préférence, le catalyseur (et le support utilisé pour la préparation du catalyseur) sont sous forme d'extrudés de diamètre compris entre 0,5 et 10 mm, de préférence entre 0,8 et 3,2 mm et de manière très préférée entre 1,0 et 2,5 mm et de longueur comprise entre 0,5 et 20 mm. On entend par « diamètre» des extrudés le diamètre du cercle circonscrit à la section droite de ces extrudés. Le catalyseur peut être avantageusement présenté sous la forme d'extrudés cylindriques, multilobés, trilobés ou quadrilobés. De préférence sa forme sera trilobée ou quadrilobée. La forme des lobes pourra être ajustée selon toutes les méthodes connues de l'art antérieur.

### 3. Support

Les caractéristiques de l'alumine, mentionnées dans cette section, correspondent aux caractéristiques de l'alumine avant imprégnation de la phase active de nickel, i.e. le support d'alumine obtenu à l'issue de l'étape c) du procédé de préparation du catalyseur selon l'invention.

Selon l'invention, le support est une alumine c'est-à-dire que le support comporte au moins 95%, de préférence au moins 98%, et de manière particulièrement préférée au moins 99% poids d'alumine par rapport au poids du support. L'alumine présente généralement une structure cristallographique du type alumine delta, gamma ou thêta, seule ou en mélange.

Selon l'invention, le support d'alumine, peut comprendre des impuretés telles que les oxydes de métaux des groupes IIA, IIIB, IVB, IIB, IIIA, IVA selon la classification CAS, de préférence la silice, le dioxyde de titane, le dioxyde de zirconium, l'oxyde de zinc, l'oxyde de magnésium et l'oxyde de calcium, ou encore des métaux alcalins, de préférence le lithium, le sodium ou le potassium, et/ou les alcalino-terreux, de préférence le magnésium, le calcium, le strontium ou le baryum ou encore du soufre.

Avantageusement, la teneur en soufre du support d'alumine est comprise entre 0,001% et 2% poids par rapport au poids total du support d'alumine, et la teneur en sodium dudit support d'alumine est comprise entre 0,001% et 2% poids par rapport au poids total dudit gel d'alumine.

La surface spécifique de l'alumine est généralement comprise entre 10 m²/g et 250 m²/g, de préférence entre 30 m²/g et 200 m²/g, de façon plus préférée entre 50 m²/g et 150m²/g.

Le volume poreux de l'alumine est généralement compris entre 0,1 ml/g et 1,2 ml/g, de préférence compris entre 0,3 ml/g et 0,9 ml/g, et de manière très préférée compris entre 0,5 ml/g et 0,9 ml/g.

### Procédé de préparation du catalyseur

Un autre objet selon l'invention concerne un procédé de préparation d'un catalyseur selon l'invention comprenant au moins les étapes suivantes :
a) on approvisionne un gel d'alumine ;
b) on met en forme le gel d'alumine de l'étape a) ;
c) on soumet le gel d'alumine mis en forme obtenu à l'issue de l'étape b) à un traitement thermique comprenant au moins une étape de traitement hydrothermal dans un autoclave en présence d'une solution acide, à une température comprise entre 100 et 800°C, et au moins une étape de calcination, à une température comprise entre 400 et 1500°C, réalisée après l'étape de traitement hydrothermal, pour obtenir un support d'alumine ;
d) on réalise l'enchaînement des étapes suivantes :
   d1) on met en contact le support d'alumine avec au moins un précurseur de la phase active de nickel pour obtenir un précurseur de catalyseur,
   d2) on sèche le précurseur de catalyseur obtenu à l'issue de l'étape d1) à une température inférieure à 250°C ;
   d2') optionnellement, on réalise un traitement thermique du précurseur de catalyseur séché obtenu à l'issue de l'étape d2) à une température comprise entre 250 et 1000°C pour obtenir un précurseur de catalyseur calciné ;
   d3) on met en contact le précurseur de catalyseur séché obtenu à l'issue de l'étape d2) (éventuellement d2') avec au moins une solution contenant au moins un additif organique choisi parmi les aldéhydes renfermant 1 à 14 atomes de carbone par molécule, les cétones ou polycétones renfermant 3 à 18 atomes de carbone par molécule, les éthers et les esters renfermant 2 à 14 atomes de carbone par molécule, les alcools ou polyalcools renfermant 1 à 14 atomes de carbone par molécule et les acides carboxyliques ou polyacides carboxyliques renfermant 1 à 14 atomes de carbone par molécule, le ratio molaire entre l'additif organique et le nickel étant supérieur à 0,05 mol/mol ;
   d4) on réalise un traitement hydrothermal du précurseur de catalyseur obtenu à l'issue de l'étape d3) à une température comprise entre 100 et 200°C pendant une durée comprise entre 30 minutes et 5 heures sous flux gazeux comprenant entre 5 et 650 grammes d'eau par kg de gaz sec ;
   d5) optionnellement, on réalise une étape de séchage entre 50 et 200°C du précurseur de catalyseur obtenu à l'issue de l'étape d4) sous flux gazeux comprenant une quantité d'eau inférieure strictement à 5 grammes d'eau par kilogramme de gaz sec ;
e) on réalise l'enchaînement des étapes suivantes :
   e1) on met en contact le support d'alumine avec au moins une solution contenant au moins un précurseur de cuivre et un précurseur de nickel à une concentration en nickel voulue pour obtenir sur le catalyseur final une teneur comprise entre 0,5 et 15 % poids en élément nickel par rapport au poids total du catalyseur final ;
   e2) on réalise au moins une étape de séchage du précurseur de catalyseur obtenu à l'issue de l'étape e1) à une température inférieure à 250°C ;
   e3) optionnellement, on réalise un traitement thermique du précurseur de catalyseur obtenu à l'issue de l'étape e2) à une température comprise entre 250 et 1000°C, en présence ou non d'eau ;
   les étapes d) et e) étant réalisées séparément dans un ordre indifférent,
f) on met en contact le support d'alumine avec au moins une solution contenant au moins un composé organique comprenant au moins une fonction acide carboxylique, ou au moins une fonction alcool, ou au moins une fonction ester, ou au moins une fonction amide, ou au moins une fonction amine,
   l'étape f) étant réalisée, soit en même temps que la sous-étape d1) de l'étape d), soit avant soit après l'étape d), mais avant l'étape g), étant entendu que lorsque l'étape f) est réalisée avant ou après l'étape d), alors ladite étape f) inclut un séchage du précurseur de catalyseur à une température inférieure à 250°C après la mise en contact du support avec ladite solution comprenant au moins un composé organique;
g) on réduit le précurseur de catalyseur issu des étapes à) à f) par mise en contact dudit précurseur de catalyseur avec un gaz réducteur à une température supérieure ou égale à 150°C et inférieure à 250°C.

Des étapes intermédiaires peuvent s'intercaler (notamment des étapes de séchages supplémentaires) et certaines étapes peuvent être effectuées plusieurs fois de suite (par exemple l'étape d1). Enfin, il est possible d'ajouter des étapes supplémentaires avant utilisation du catalyseur à l'issue de l'étape g).

De préférence, on réalise une étape de séchage puis de calcination à l'issue de l'étape b) de mise en forme (mais avant la réalisation de l'étape c).

De préférence, les étapes les étapes d2') et d5) ne sont pas optionnelles.

Les étapes a) à g) dudit procédé de préparation sont décrites en détail ci-après.

### Etape a) - Gel d'alumine

Le catalyseur selon l'invention comprend un support alumine qui est obtenu à partir d'une alumine gel (ou gel d'alumine) qui comprend essentiellement un précurseur du type oxy(hydroxyde) d'aluminium (AIO(OH)) - également dénommé boehmite.

Selon l'invention, le gel d'alumine (ou autrement dénommé gel de boehmite) est synthétisé par précipitation de solutions basiques et/ou acides de sels d'aluminium induite par changement de pH ou tout autre méthode connue de l'Homme de métier (P. Euzen, P. Raybaud, X. Krokidis, H. Toulhoat, J.L. Le Loarer, J.P. Jolivet, C. Froidefond, Alumina, in Handbook of Porous Solids, Eds F. Schüth, K.S.W. Sing, J. Weitkamp, Wiley-VCH, Weinheim, Germany, 2002, pp. 1591-1677).

Généralement la réaction de précipitation est effectuée à une température comprise entre 5°C et 80°C et à un pH compris entre 6 et 10. De manière préférée la température est comprise entre 35°C et 70°C et le pH est compris entre 6 et 10.

Selon un mode de réalisation, l'alumine gel est obtenue par mise en contact d'une solution aqueuse d'un sel acide d'aluminium avec une solution basique. Par exemple le sel acide d'aluminium est choisi dans le groupe constitué par le sulfate d'aluminium, le nitrate d'aluminium ou le chlorure d'aluminium et de manière préférée, ledit sel acide est le sulfate d'aluminium. La solution basique est préférentiellement choisi parmi la soude ou la potasse. Alternativement, on peut mettre en contact une solution alcaline de sels d'aluminium qui peuvent être choisis dans le groupe constitué par l'aluminate de sodium et l'aluminate de potassium avec une solution acide. Dans une variante très préférée, le gel est obtenu par mise en contact d'une solution d'aluminate de sodium avec de l'acide nitrique. La solution d'aluminate de sodium présente avantageusement une concentration comprise entre 10⁻⁵ et 10⁻¹ mol.L⁻¹ et de manière préférée cette concentration est comprise entre 10⁻⁴ et 10⁻² mol.L⁻¹. Selon un autre mode de réalisation, l'alumine gel est obtenue par mise en contact d'une solution aqueuse de sels acides d'aluminium avec une solution alcaline de sels d'aluminium.

### Etape b) - Mise en forme du support

Le support peut avantageusement être mis en forme par toute technique connue de l'Homme du métier. La mise en forme peut être réalisée par exemple par malaxage-extrusion, par pastillage, par la méthode de la coagulation en goutte (oil-drop), par granulation au plateau tournant ou par toute autre méthode bien connue de l'homme du métier. Les catalyseurs selon l'invention peuvent éventuellement être fabriqués et employés sous la forme d'extrudés, de tablettes, de billes. La méthode de mise en forme avantageuse selon l'invention est l'extrusion et les formes d'extrudés préférées sont cylindriques, cylindriques torsadées ou multilobées (2, 3, 4 ou 5 lobes par exemple).

Dans un mode de réalisation particulier, le gel d'alumine obtenu à l'issue de l'étape a) est soumis à une étape de malaxage de préférence dans un milieu acide. L'acide mis en oeuvre peut être par exemple de l'acide nitrique. Cette étape est réalisée au moyen d'outils connus tels que des malaxeurs bras en Z, des malaxeurs à meules, des mono ou bi-vis continues permettant la transformation du gel en un produit ayant la consistance d'une pâte. Selon un mode de réalisation avantageux, on apporte un ou plusieurs composés dits "agents porogènes" dans le milieu de malaxage. Ces composés présentent la propriété de se dégrader par chauffage et créer ainsi une porosité dans le support. Par exemple on peut utiliser comme composés porogènes la farine de bois, le charbon de bois, des goudrons, des matières plastiques. La pâte ainsi obtenue après malaxage est passée au travers d'une filière d'extrusion. Généralement les extrudés ont un diamètre compris 0,5 et 10 mm, de préférence entre 0,8 et 3,2 mm et de manière très préférée entre 1,0 et 2,5 mm et de longueur comprise entre 0,5 et 20 mm. Ces extrudés peuvent être de forme cylindrique, multilobée (par exemple trilobée ou quadrilobée).

Après sa mise en forme, le support est éventuellement séché avant de subir le traitement hydrothermal selon l'étape c) du procédé. Par exemple le séchage est effectué à une température comprise entre 50 et 200°C. Le support séché est éventuellement calciné avant de subir le traitement hydrothermal selon l'étape c) du procédé. Par exemple, la calcination est effectuée à une température comprise entre 200 et 1000°C, en présence ou non d'un flux d'air contenant jusqu'à 150 d'eau par kilogramme d'air sec.

### Etape c) - Traitement thermique

Le support obtenu à l'issue de l'étape b) subit ensuite une étape de traitement thermique qui permet de lui conférer des propriétés physiques répondant à l'application envisagée.

On désigne par le terme "traitement hydrothermal", un traitement par passage en autoclave en présence d'eau à une température supérieure à la température ambiante.

Au cours de ce traitement hydrothermal, on peut traiter de différentes manières l'alumine mise en forme. Ainsi, on peut imprégner l'alumine d'une solution acide, préalablement à son passage à l'autoclave, le traitement hydrothermal de l'alumine pouvant être fait soit en phase vapeur, soit en phase liquide, cette phase vapeur ou liquide de l'autoclave pouvant être acide ou non. Cette imprégnation, avant le traitement hydrothermal, peut être effectuée à sec ou par immersion de l'alumine dans une solution aqueuse acide. Par imprégnation à sec, on entend une mise en contact de l'alumine avec un volume de solution inférieur ou égal au volume poreux total de l'alumine traitée. De préférence, l'imprégnation est réalisée à sec.

On peut également traiter le support extrudé sans imprégnation préalable par une solution acide, l'acidité étant dans ce cas apportée par le liquide aqueux de l'autoclave.

La solution aqueuse acide comprend au moins un composé acide permettant de dissoudre au moins une partie de l'alumine des extrudés. On entend par "composé acide permettant de dissoudre au moins une partie de l'alumine des extrudés", tout composé acide qui, mis en contact avec les extrudés d'alumine, réalise la mise en solution d'au moins une partie des ions aluminium. L'acide doit, de préférence, dissoudre au moins 0,5 % en poids d'alumine des extrudés d'alumine.

De préférence, cet acide est choisi parmi les acides forts tels que l'acide nitrique, l'acide chlorhydrique, l'acide perchlorique, l'acide sulfurique ou un acide faible mis en oeuvre à une concentration telle que sa solution aqueuse présente un pH inférieur à 4, tel que l'acide acétique, ou un mélange de ces acides.

Selon un mode préféré, on réalise le traitement hydrothermal en présence d'acide nitrique et d'acide acétique pris seul ou en mélange. L'autoclave est de préférence un autoclave à panier rotatif tel que celui défini dans la demande de brevet EP-A-0 387 109.

Le traitement hydrothermal peut également être réalisé sous pression de vapeur saturante ou sous une pression partielle de vapeur d'eau au moins égale à 70 % de la pression de vapeur saturante correspondant à la température de traitement.

De préférence le traitement hydrothermal est conduit à une température comprise entre 100 et 800°C, de préférence entre 200 et 700°C, de préférence entre 30 minutes et 8 heures, plus préférentiellement entre 30 minutes et 3 heures.

De préférence, l'étape de calcination qui a lieu après le traitement hydrothermal par passage en autoclave se déroule à une température généralement comprise entre 400 et 1500°C, de préférence entre 800 et 1300°C, de préférence pendant 1 et 5 heures sous air dont la teneur en eau est généralement comprise entre 0 et 700 g d'eau par kilogramme d'air sec.

A l'issue de l'étape c), l'alumine obtenue présente les propriétés texturales spécifiques telles que décrites ci-avant.

### Etape d)

L'étape d) comprend les sous-étapes suivantes.

### Etape d1) - Mise en contact du support avec un précurseur de la phase active de nickel

La mise en contact du support avec une solution contenant un précurseur de la phase active de nickel, conformément à la mise en oeuvre de l'étape d1), peut être réalisée par imprégnation, à sec ou en excès, ou encore par dépôt - précipitation, selon des méthodes bien connues de l'Homme du métier.

Ladite étape d1) est préférentiellement réalisée par imprégnation du support consistant par exemple en la mise en contact du support avec au moins une solution aqueuse contenant un précurseur de nickel. Le pH de ladite solution pourra être modifié par l'ajout éventuel d'un acide ou d'une base.

De manière préférée, ladite étape d1) est réalisée par imprégnation à sec, laquelle consiste à mettre en contact le support avec au moins une solution, contenant, de préférence constituée de, au moins un précurseur du nickel, dont le volume de la solution est compris entre 0,25 et 1,5 fois le volume poreux du support à imprégner.

De manière préférée, ledit précurseur de nickel est introduit en solution aqueuse, par exemple sous forme de nitrate, de carbonate, d'acétate, de chlorure, d'oxalate, de complexes formés par un polyacide ou un acide-alcool et ses sels, de complexes formés avec les acétylacétonates, ou de tout autre dérivé inorganique soluble en solution aqueuse, laquelle est mise en contact avec ledit support. De manière préférée, on utilise avantageusement comme précurseur de nickel, le nitrate de nickel, le chlorure de nickel, l'acétate de nickel ou le hydroxycarbonate de nickel. De manière très préférée, le précurseur de nickel est le nitrate de nickel.

Selon une autre variante, la solution aqueuse peut contenir de l'ammoniaque ou des ions ammonium NH₄⁺.

La concentration en nickel en solution est ajustée selon le type imprégnation (imprégnation à sec ou en excès) et le volume poreux du support de façon à obtenir pour le catalyseur supporté, une teneur en nickel comprise entre 1 et 50 % poids en élément nickel par rapport au poids total du catalyseur, plus préférentiellement entre 2 et 40 % poids et encore plus préférentiellement entre 3 et 35 % poids et encore plus préférentiellement 5 et 25 % poids.

### Etape d2) - Séchage

L'étape de séchage est effectuée sous flux gazeux comprenant une quantité d'eau inférieure à 150 grammes d'eau par kilogramme de gaz sec, de préférence inférieure à 50 g d'eau par kilogramme de gaz sec, à une température inférieure à 250°C, de préférence comprise entre 15 et 240°C, plus préférentiellement entre 30 et 220°C, encore plus préférentiellement entre 50 et 200°C, et de manière encore plus préférentielle entre 70 et 180°C, pendant une durée typiquement comprise entre 10 minutes et 24 heures. Des durées plus longues ne sont pas exclues, mais n'apportent pas nécessairement d'amélioration.

Le gaz peut contenir de l'oxygène, de l'azote ou un gaz inerte et de préférence le gaz est l'air.

### Etape d2') Calcination (optionnelle)

L'étape de calcination optionnelle est effectuée sous flux gazeux comprenant une quantité d'eau inférieure à 150 grammes d'eau par kilogramme de gaz sec, de préférence inférieure à 50 g d'eau par kilogramme de gaz sec, à une température comprise entre 250°C et 1000°C, de préférence entre 250 et 750°C. La durée de ce traitement thermique est généralement comprise entre 15 minutes et 10 heures. Des durées plus longues ne sont pas exclues, mais n'apportent pas nécessairement d'amélioration.

Le gaz peut contenir de l'oxygène, de l'azote ou un gaz inerte et de préférence le gaz est de l'air.

A l'issue des étapes d2) ou d2'), le nickel est réparti de façon homogène sur le support.

### Etape d3) - Additif

Selon l'étape d3) du procédé de préparation du catalyseur, on met en contact le précurseur de catalyseur obtenu à l'issue de l'étape d2), éventuellement à l'issue de l'étape d2'), avec au moins une solution comprenant au moins un additif organique choisi parmi les aldéhydes renfermant de 1 à 14 atomes de carbone par molécule (de préférence de 2 à 12), les cétones ou polycétones renfermant de 3 à 18 (de préférence de 3 à 12) atomes de carbone par molécule, les éthers ou les esters renfermant de 2 à 14 (de préférence de 3 à 12) atomes de carbone par molécule, les alcools ou polyalcools renfermant de 1 à 14 (de préférence de 2 à 12) atomes de carbone par molécule et les acides carboxyliques ou polyacides carboxyliques renfermant de 1 à 14 (de préférence de 1 à 12) atomes de carbone par molécule. L'additif organique peut être composé d'une combinaison des différents groupes fonctionnels cités ci-dessus.

De préférence, l'additif organique est choisi parmi l'acide formique HCOOH, le formaldéhyde CH₂O, l'acide acétique CH₃COOH, l'acide citrique, l'acide oxalique, l'acide glycolique (HOOC-CH₂-OH), l'acide malonique (HOOC-CH₂-COOH), l'éthanol, le méthanol, le formiate d'éthyle HCOOC₂H₅, le formiate de méthyle HCOOCH₃, le paraldéhyde (CH₃-CHO)₃, l'acétaldéhyde C₂H₄O, l'acide gamma-valérolactone (C₅H₈O₂), le glucose, le sorbitol et le trioxane.

De manière particulièrement préférée, l'additif organique est l'acide formique.

Il est essentiel que l'étape d'ajout de l'additif organique sur le catalyseur (étape d3)) soit réalisée après l'étape de mise en contact du support avec le précurseur de la phase active de nickel.

De manière préférée, ladite étape d3) est réalisée par imprégnation du précurseur de catalyseur obtenu à l'issue de la mise en oeuvre de l'étape d2) ou de l'étape d2'), avec une solution comprenant au moins un additif organique tel que cité ci-avant. L'imprégnation est généralement effectuée en solution aqueuse ou en solution organique ou en suspension dans la solution aqueuse ou organique, de préférence en solution aqueuse. Lorsque l'on opère en solution ou suspension organique, on utilisera à titre de solvant organique de préférence un alcool ou polyalcool, glycol ou polyglycol.

De manière préférée, ladite étape d3) est réalisée par imprégnation à sec, laquelle consiste à mettre en contact le précurseur de catalyseur obtenu à l'issue de la mise en oeuvre de l'étape d2) ou de l'étape d2'), avec une solution comprenant au moins un additif organique tel que cité ci-avant, dont le volume de la solution est compris entre 0,25 et 1,5 fois le volume poreux du précurseur de catalyseur à imprégner.

L'imprégnation est généralement réalisée à une température entre 0 et 50°C, de préférence entre 10 et 40°C, et de manière particulièrement préférée à température ambiante.

Selon l'invention, le ratio molaire entre l'additif organique et le nickel est supérieur à 0,05 mol/mol, de préférence compris entre 0,1 et 5 mol/mol, plus préférentiellement compris entre 0,12 et 3 mol/mol, et de façon encore plus préférée compris entre 0,15 et 2,5 mol/mol.

### Etape d4) - Traitement hydrothermal

Selon l'étape d4) du procédé de préparation du catalyseur selon l'invention, on effectue un traitement hydrothermal du produit issu de l'étape d3) à une température comprise entre 100°C et 200°C, de préférence entre 130°C et 170°C, et plus particulièrement autour de 150°C, sous flux gazeux comprenant entre 5 et 650 grammes d'eau par kilogramme de gaz sec, de préférence entre 7 et 150 grammes d'eau par kilogramme de gaz sec, de façon encore plus préférée entre 10 et 50 grammes d'eau par kilogramme de gaz sec. Le gaz peut contenir de l'oxygène, de l'azote ou un gaz inerte et de préférence le gaz est de l'air.

La durée du traitement hydrothermal est généralement entre 30 minutes et 5 heures, de préférence entre 1 à 3 heures.

### Etape d5) - Séchage (optionnelle)

L'étape d4) peut être suivie d'une étape d5) de séchage entre 50 et 200°C sous flux gazeux comprenant une quantité d'eau inférieure strictement à 5 grammes d'eau par kilogramme de gaz sec, avantageusement pendant une durée comprise entre 30 minutes et 5 heures, de préférence entre 1 à 3 heures.

Le gaz peut contenir de l'oxygène, de l'azote ou un gaz inerte et de préférence le gaz est de l'air.

A l'issue de l'étape d4) ou éventuellement de l'étape d5), on obtient un catalyseur «semi egg-shell » tel que représenté schématiquement en figure 1 et dont les caractéristiques sont décrites ci-dessus.

### Etape e)

L'étape e) comprend les sous-étapes suivantes.

### Etape e1) Mise en contact d'un précurseur de cuivre et de nickel avec le support

Le dépôt du nickel et du cuivre sur le support d'alumine peut être réalisé par imprégnation, à sec ou en excès, ou encore par dépôt - précipitation, selon des méthodes bien connues de l'Homme du métier.

Ladite étape e1) est préférentiellement réalisée par imprégnation du précurseur de catalyseur consistant par exemple en la mise en contact dudit support avec au moins une solution, aqueuse ou organique (par exemple le méthanol ou l'éthanol ou le phénol ou l'acétone ou le toluène ou le diméthylsulfoxyde (DMSO)) ou bien constituée d'un mélange d'eau et d'au moins un solvant organique, comprenant, de préférence étant constituée de, au moins un précurseur de nickel et au moins un précurseur de cuivre au moins partiellement à l'état dissous, ou encore en la mise en contact dudit précurseur de catalyseur avec au moins une solution colloïdale comprenant, de préférence étant constituée de, au moins un précurseur du nickel et d'un précurseur de cuivre sous forme oxydées (nanoparticules d'oxyde, d'oxy(hydroxyde) ou d'hydroxyde du nickel et de cuivre) ou sous forme réduites (nanoparticules métalliques du nickel et de cuivre à l'état réduit). De préférence, la solution est aqueuse. Le pH de cette solution peut être modifié par l'ajout éventuel d'un acide ou d'une base.

De manière préférée, ladite étape e1) est réalisée par imprégnation à sec, laquelle consiste à mettre en contact le support du précurseur de catalyseur avec une solution, comprenant, de préférence constituée de, au moins un précurseur du nickel et au moins un précurseur de cuivre, dont le volume de la solution est compris entre 0,25 et 1,5 fois le volume poreux du support à imprégner.

Lorsque le précurseur de nickel est introduit en solution aqueuse, on utilise avantageusement un précurseur de nickel sous forme de nitrate, de carbonate, d'acétate, de chlorure, d'hydroxyde, d'hydroxycarbonate, d'oxalate, de sulfate, de formiate, de complexes formés par un polyacide ou un acide-alcool et ses sels, de complexes formés avec les acétylacétonates, de complexes tétrammine ou hexammine, ou encore de tout autre dérivé inorganique soluble en solution aqueuse, laquelle est mise en contact avec ledit précurseur de catalyseur. De manière préférée, on utilise avantageusement comme précurseur de nickel, le nitrate de nickel, l'hydroxyde de nickel, le carbonate de nickel, le chlorure de nickel, ou le hydroxycarbonate de nickel. De manière très préférée, le précurseur de nickel est le nitrate de nickel, le carbonate de nickel ou le hydroxyde de nickel.

Lorsque le précurseur de cuivre est introduit en solution aqueuse, on utilise avantageusement un précurseur de cuivre sous forme minérale ou organique. Sous forme minérale, le précurseur de cuivre peut être choisi parmi l'acétate de cuivre, l'acétylacétonate de cuivre, le nitrate de cuivre, le sulfate de cuivre, le chlorure de cuivre, le bromure de cuivre, l'iodure de cuivre ou le fluorure de cuivre. De manière très préférée, le sel précurseur du cuivre est le nitrate de cuivre.

Selon l'invention, le précurseur de nickel est approvisionnée à l'étape e1) à une concentration voulue pour obtenir sur le catalyseur final (i.e. obtenu à l'issue de l'étape f) de réduction ou de l'étape g) de passivation si cette dernière est effectuée) une teneur comprise entre 0,5 et 10 % poids en élément nickel par rapport au poids total du catalyseur final, de préférence entre 0,5 et 8% en poids, plus préférentiellement entre 1 et 7% en poids, encore plus préférentiellement entre 1 et 5% en poids.

Les quantités du ou des précurseurs de cuivre introduites dans la solution selon l'étape e1) sont choisies de telle manière que la teneur totale en cuivre est comprise entre 0,5 et 15 % en poids en élément cuivre par rapport au poids total du catalyseur final (i.e. obtenu à l'issue de l'étape f) de réduction ou de l'étape g) de passivation si cette dernière est effectuée), de préférence comprise entre 0,5 et 12 % poids, de manière préférée comprise entre 0,75 et 10 % poids, et encore plus préférentiellement entre 1 et 9% en poids.

### Etape e2) Séchage du support imprégné

L'étape e2) de séchage du support imprégné est effectuée à une température inférieure à 250°C, de préférence comprise entre 15 et 180°C, plus préférentiellement entre 30 et 160°C, encore plus préférentiellement entre 50 et 150°C, et de manière encore plus préférentielle entre 70 et 140°C, typiquement pendant une durée comprise entre 10 minutes et 24 heures. Des durées plus longues ne sont pas exclues, mais n'apportent pas nécessairement d'amélioration.

L'étape de séchage peut être effectuée par toute technique connue de l'Homme du métier. Elle est avantageusement effectuée sous une atmosphère inerte ou sous une atmosphère contenant de l'oxygène ou sous un mélange de gaz inerte et d'oxygène. Elle est avantageusement effectuée à pression atmosphérique ou à pression réduite. De manière préférée, cette étape est réalisée à pression atmosphérique et en présence d'air ou d'azote.

### Etape e3) Traitement thermique du catalyseur séché (étape optionnelle)

Le précurseur de catalyseur séché peut subir une étape complémentaire de traitement thermique, avant l'étape f) de réduction, à une température comprise entre 250 et 1000°C et de préférence entre 250 et 750°C, typiquement pendant une durée comprise entre 15 minutes et 10 heures, sous une atmosphère inerte ou sous une atmosphère contenant de l'oxygène, en présence d'eau ou non. Des durées de traitement plus longues ne sont pas exclues, mais n'apportent pas nécessaire d'amélioration.

On entend par « traitement thermique » le traitement en température respectivement sans présence ou en présence d'eau. Dans ce dernier cas, le contact avec la vapeur d'eau peut se dérouler à pression atmosphérique ou en pression autogène. Plusieurs cycles combinés sans présence ou avec présence d'eau peuvent être réalisés. Après ce ou ces traitement(s), le précurseur de catalyseur comprend du nickel sous forme oxyde, c'est-à-dire sous forme NiO.

En cas de présence d'eau, la teneur en eau est de préférence comprise entre 150 et 900 grammes par kilogramme d'air sec, et de manière encore plus préférée, entre 250 et 650 grammes par kilogramme d'air sec.

### Mise en oeuvre de l'étape e) par rapport aux autres étapes du procédé de préparation

Le procédé de préparation du catalyseur au nickel comporte plusieurs modes de mises en oeuvre. Ils se distinguent notamment par l'ordre d'introduction des précurseurs de nickel et de cuivre constituant l'alliage NiCu. La mise en contact des précurseurs de nickel et de cuivre avec le support pouvant être effectuée soit après la mise en contact du précurseur de nickel avec le support, soit avant la mise en contact du précurseur de nickel avec le support. Un premier mode de mise en oeuvre consiste à effectuer ladite étape e) préalablement à ladite étape d).

Un deuxième mode de mise en oeuvre consiste à effectuer ladite étape d) préalablement à ladite étape e).

Lorsque l'étape e) est réalisée avant ou après l'étape d), ladite étape e) inclut un séchage du précurseur de catalyseur à une température inférieure à 250°C après la mise en contact du support avec ladite solution comprenant au moins un composé organique.

### Etape f) Ajout du composé organique

La mise en contact dudit support avec au moins une solution contenant au moins un composé organique comprenant au moins une fonction acide carboxylique, ou au moins une fonction alcool, ou au moins une fonction ester, ou au moins une fonction amide, ou au moins une fonction amine conformément à la mise en oeuvre de ladite étape f), peut être réalisé par toute méthode bien connue de l'Homme du métier. En effet, il a été en outre remarqué que les catalyseurs selon l'invention préparés en présence d'un composé organique (parmi ceux cités ci-après) sont plus actifs que les catalyseurs préparés en l'absence de ce type de composé organique. Cet effet est lié à la diminution de la taille des particules de nickel.

En particulier, ladite étape f) peut être réalisée par imprégnation, à sec ou en excès selon des méthodes bien connues de l'Homme du métier. De manière préférée, ladite étape f) est réalisée par imprégnation à sec, laquelle consiste à mettre en contact le support du catalyseur avec un volume de ladite solution compris entre 0,25 et 1,5 fois le volume poreux du support à imprégner.

Ladite solution contenant au moins un composé organique comprenant au moins une fonction acide carboxylique, ou au moins une fonction alcool, ou au moins une fonction ester, ou au moins une fonction amide ou au moins une fonction amine, peut être aqueuse ou organique (par exemple le méthanol ou l'éthanol ou le phénol ou l'acétone ou le toluène ou le diméthylsulfoxyde (DMSO)) ou bien constituée d'un mélange d'eau et d'au moins un solvant organique. Ledit composé organique est préalablement au moins partiellement dissous dans ladite solution à la concentration voulue. De préférence, ladite solution est aqueuse ou contient de l'éthanol. De façon encore plus préférée, ladite solution est aqueuse. Le pH de ladite solution pourra être modifié par l'ajout éventuel d'un acide ou d'une base. Dans un autre mode de réalisation possible, le solvant peut être absent de la solution d'imprégnation.

Dans le mode de réalisation dans lequel l'étape f) est réalisée par imprégnation, à sec ou en excès, de préférence à sec, l'imprégnation du support avec au moins une solution contenant au moins ledit composé organique peut être avantageusement réalisée via au moins deux cycles d'imprégnation, en utilisant des composés organiques identiques ou différents à chaque cycle. Dans ce cas, chaque imprégnation est avantageusement suivie d'un séchage et éventuellement d'un traitement thermique.

Avantageusement, le rapport molaire entre ledit composé organique introduit à l'étape f) et l'élément nickel également introduit à l'étape d1) est compris entre 0,01 et 5,0 mol/mol, de préférence entre 0,05 et 2,0 mol/mol, plus préférentiellement entre 0,1 et 1,5 mol/mol et encore plus préférentiellement entre 0,3 et 1,2 mol/mol.

Le composé organique selon l'étape f) peut comprendre au sein de la même molécule plusieurs fonctions organiques acides carboxylique, alcools esters, amides ou amines, identiques ou différentes. Le composé organique selon l'étape f) peut comprendre une combinaison de plusieurs fonctions organiques choisies parmi les fonctions organiques de acides carboxylique, alcools esters, amides ou amines.

De préférence, le composé organique de l'étape f) est différent de l'additif organique de l'étape d2).

### A) Composé organique comprenant au moins une fonction acide carboxylique

Dans un mode de réalisation selon l'invention, le composé organique comprend au moins une fonction acide carboxylique.

Ledit composé organique comprenant au moins une fonction acide carboxylique peut être un composé organique aliphatique, saturé ou insaturé, ou un composé organique aromatique. De préférence, le composé organique aliphatique, saturé ou insaturé, comprend entre 1 et 9 atomes de carbone, de préférence entre 2 et 7 atomes de carbone. De préférence, le composé organique aromatique comprend entre 7 et 10 atomes de carbone, de préférence entre 7 et 9 atomes de carbone.

Ledit composé organique aliphatique, saturé ou insaturé, ou ledit composé organique aromatique, comprenant au moins une fonction acide carboxylique peut être choisi parmi les acides monocarboxyliques, les acides dicarboxyliques, les acides tricarboxyliques, les acides tétracarboxyliques.

Avantageusement, le composé organique comprenant au moins une fonction acide carboxylique est choisi parmi l'acide éthanedioïque (acide oxalique), l'acide propanedioïque (acide malonique), l'acide pentanedioïque (acide glutarique), l'acide hydroxyacétique (acide glycolique), l'acide 2-hydroxypropanoïque (acide lactique), l'acide 2-hydroxypropanedioïque (acide tartronique), l'acide 2-hydroxypropane-1,2,3-tricarboxylique (acide citrique), l'acide 2,3-dihydroxybutanedioïque (acide tartrique), l'acide 2-oxopropanoïque (acide pyruvique), l'acide 4-oxopentanoïque (acide lévulinique).

### B) Composé organique comprenant au moins une fonction alcool

Dans un autre mode de réalisation selon l'invention, le composé organique comprend au moins une fonction alcool.

De préférence, ledit composé organique comprend entre 2 et 20 atomes de carbone, de préférence entre 2 et 12 atomes de carbone, et encore plus préférentiellement entre 2 et 8 atomes de carbone.

Avantageusement, le composé organique est choisi parmi le méthanol, l'éthanol, le phénol, l'éthylène glycol, le propane-1,3-diol, le butane-1,4-diol, le pentane-1,5-diol, l'hexane-1,6-diol, le glycérol, le xylitol, le mannitol, le sorbitol, le pyrocatéchol, le résorcinol, l'hydroquinol, le diéthylène glycol, le triéthylène glycol, les polyéthylène glycol ayant une masse molaire moyenne inférieure à 600 g/mol, le glucose, le mannose, le fructose, le sucrose, le maltose, le lactose, sous l'une quelconque de leurs formes isomères.

### C) Composé organique comprenant au moins une fonction ester

Dans un autre mode de réalisation selon l'invention, le composé organique comprend au moins une fonction ester. De préférence, ledit composé organique comprend entre 2 et 20 atomes de carbone, de préférence entre 3 et 14 atomes de carbone, et encore plus préférentiellement entre 3 et 8 atomes de carbone.

Ledit composé organique peut être choisi parmi un ester d'acide carboxylique, linéaire ou cyclique ou cyclique insaturé, ou un ester d'acide carbonique, cyclique ou linéaire ou encore un diester d'acide carbonique linéaire. Dans le cas d'un ester cyclique d'acide carboxylique, ledit composé est la γ-valérolactone.

Dans le cas d'un ester cyclique insaturé (contenant des insaturations dans le cycle) d'acide carboxylique, le composé peut être le furane ou la pyrone ou l'un quelconque de leurs dérivés, tel que la 6-pentyl-α-pyrone.

Dans le cas d'un ester linéaire d'acide carboxylique, le composé peut être un composé comportant une seule fonction d'ester répondant à la formule brute RCOOR', dans laquelle R et R' sont des groupements alkyles, linéaires, ramifiés, ou cycliques, ou des groupements alkyles contenant des insaturations, ou des groupements alkyles substitués par un ou plusieurs cycles aromatiques, ou des groupements aryles, contenant chacun entre 1 et 15 atomes de carbone, et pouvant être identiques ou différents. Le groupement R peut aussi être l'atome d'hydrogène H. Ledit composé organique est de préférence le laurate de méthyle.

Dans un autre mode de réalisation selon l'invention, le composé organique peut être un composé comportant au moins deux fonctions esters d'acide carboxylique. De préférence, ledit composé est le succinate de diméthyle.

Dans un autre mode de réalisation selon l'invention, le composé organique peut être un composé comportant au moins une fonction ester d'acide carboxylique et au moins un deuxième groupe fonctionnel choisi parmi les alcools, les éthers, les cétones, les aldéhydes. De préférence, ledit composé est le malate de diméthyle.

Avantageusement, ledit composé organique comprend au moins une fonction ester d'acide carboxylique et au moins une fonction cétone ou aldéhyde. Dans le cas d'un ester cyclique d'acide carbonique, le composé est le carbonate de propylène. Dans le cas d'un ester linéaire d'acide carbonique, le composé est choisi parmi le carbonate de diméthyle, le carbonate de diéthyle ou le carbonate de diphényle. Dans le cas d'un diester linéaire d'acide carbonique, le composé est choisi parmi le dicarbonate de diméthyle, le dicarbonate de diéthyle, le dicarbonate de di-tert-butyle.

### D) Composé organique comprenant au moins une fonction amide

Dans un autre mode de réalisation selon l'invention, le composé organique comprend au moins une fonction amide, choisie parmi une fonction amide acyclique ou une fonction amide cyclique, comprenant éventuellement des substituants alkyles ou aryles ou alkyles contenant des insaturations. Les fonctions amides peuvent être choisies parmi les amides primaires, secondaires ou tertiaires.

Avantageusement, le composé organique comprenant au moins une fonction amide est choisi parmi la formamide, la N-méthylformamide, la N,N-diméthylformamide, la N-éthylformamide, la N,N-diéthylformamide, l'acétamide, la N-méthylacétamide, la N,N-diméthylméthanamide, la N,N-diéthylacétamide, la N,N-diméthylpropionamide, la propanamide, la 2-pyrrolidone, la N-méthyl-2-pyrrolidone, la γ-lactame, la caprolactame, l'acétylleucine, l'acide N-acétylaspartique, l'acide aminohippurique, l'acide N-acétylglutamique, l'acide 4-acétamidobenzoïque, la lactamide et la glycolamide, l'urée, la N-méthylurée, la N,N'-diméthylurée, la 1,1-diméthylurée, la tétraméthylurée selon l'une quelconque de leurs formes isomères.

### E) Composé organique comprenant au moins une fonction amine

Dans un autre mode de réalisation selon l'invention, le composé organique comprend au moins une fonction amine. Ledit composé organique comprend entre 1 et 20 atomes de carbone, de préférence entre 1 et 14 atomes de carbone, et encore plus préférentiellement entre 2 et 8 atomes de carbone.

Dans un mode de réalisation selon l'invention, ledit composé organique comprenant au moins une fonction amine répondant à la formule brute CₓN_{y}H_{z} dans laquelle 1 ≤ x ≤ 20, 1 ≤ y ≤ x, 2 ≤ z ≤ 2x+2. Plus particulièrement, le composé organique est choisi parmi l'éthylènediamine, le diaminohexane, la tétraméthylènediamine, l'hexaméthylènediamine, la tétraméthyléthylènediamine, la tétraéthyléthylènediamine, la diéthylènetriamine, la triéthylènetétramine.

Dans un mode de réalisation selon l'invention, ledit composé organique comprend au moins une fonction amine et au moins une fonction acide carboxylique (acide aminé). Lorsque le composé est un acide aminé, il est de préférence choisi parmi l'alanine, l'arginine, la lysine, la proline, la sérine, la thréonine, l'EDTA.

Parmi tous les modes de réalisation ci-avant, le composé organique est choisi parmi l'acide oxalique, l'acide malonique, l'acide glycolique, l'acide acide lactique, l'acide tartronique, l'acide citrique, l'acide tartrique, l'acide pyruvique, l'acide lévulinique, l'éthylène glycol, le propane-1,3-diol, le butane-1,4-diol, le glycérol, le xylitol, le mannitol, le sorbitol, le diéthylène glycol, le glucose, la gamma valérolactone, le carbonate de diméthyle, le carbonate de diéthyle, la formamide, la N-méthylformamide, l'acétamide, la N-méthylacétamide, la N,N-diméthylméthanamide, la 2-pyrrolidone, la γ-lactame, la lactamide, l'urée, l'alanine, l'arginine, la lysine, la proline, la sérine, l'EDTA.

### Mise en oeuvre de l'étape f) par rapport aux autres étapes du procédé de préparation

Le procédé de préparation du catalyseur au nickel comporte plusieurs modes de mises en oeuvre. Ils se distinguent notamment par l'ordre d'introduction du composé organique et du précurseur de nickel, la mise en contact du composé organique avec le support pouvant être effectuée soit après la mise en contact du précurseur de nickel avec le support, soit avant la mise en contact du précurseur de nickel avec le support, ou soit en même temps que la mise en contact du nickel avec le support.

Un premier mode de mise en oeuvre consiste à effectuer ladite étape d) préalablement à ladite étape f).

Un deuxième mode de mise en oeuvre consiste à effectuer ladite étape f) préalablement à ladite étape d).

Lorsque l'étape f) est réalisée avant ou après l'étape d), ladite étape f) inclut un séchage du précurseur de catalyseur à une température inférieure à 250°C après la mise en contact du support avec ladite solution comprenant au moins un composé organique.

Chaque étape de mise en contact du support avec le précurseur de nickel (étape d1), et de mise en contact du support avec au moins une solution contenant au moins un composé organique comprenant au moins une fonction acide carboxylique, ou au moins une fonction alcool, ou au moins une fonction ester, ou au moins une fonction amide ou au moins une fonction amine, (étape f), est réalisée au moins une fois et peut avantageusement être réalisée plusieurs fois, éventuellement en présence d'un précurseur de nickel et/ou d'un composé organique identique(s) ou différent(s) à chaque étape d1) et/ou f) respectivement.

Un troisième mode de mise en oeuvre consiste à effectuer ladite étape d1) et ladite étape f) simultanément (co-contactage). Ce mode de mise en oeuvre peut comprendre avantageusement la mise en oeuvre d'une ou plusieurs étapes d1), éventuellement avec un précurseur de nickel identique ou différent à chaque étape d1). En particulier, une ou plusieurs étapes d1) précède(nt) et/ou suive(nt) avantageusement ladite étape de co-contactage, éventuellement avec un précurseur de nickel identique ou différent à chaque étape. Ce mode de mise en oeuvre peut également comprendre plusieurs étapes de co-contactage : les étapes d1) et f) sont effectuées de manière simultanée à plusieurs reprises, éventuellement en présence d'un précurseur de nickel et/ou d'un composé organique identique(s) ou différent(s) à chaque étape de co-contactage.

Chaque étape de mise en contact peut être de préférence suivie d'une étape de séchage intermédiaire. L'étape de séchage intermédiaire est effectuée à une température inférieure à 250°C, de préférence comprise entre 15 et 240°C, plus préférentiellement entre 30 et 220°C, encore plus préférentiellement entre 50 et 200°C, et de manière encore plus préférentielle entre 70 et 180°C. Avantageusement, lorsqu'on réalise une étape de séchage intermédiaire, on peut réaliser une étape de calcination intermédiaire. L'étape de calcination intermédiaire est effectuée à une température comprise entre 250°C et 1000°C, de préférence entre 250 et 750°C.

Avantageusement, après chaque étape de mise en contact, que ce soit une étape de mise en contact du précurseur de nickel avec le support, une étape de mise en contact du composé organique avec le support, ou une étape de mise en contact du précurseur de nickel et du composé organique de manière simultanée avec le support, on peut laisser maturer le support imprégné, éventuellement avant une étape de séchage intermédiaire. La maturation permet à la solution de se répartir de manière homogène au sein du support. Lorsqu'une étape de maturation est réalisée, ladite étape est opérée avantageusement à pression atmosphérique ou à pression réduite, sous une atmosphère inerte ou sous une atmosphère contenant de l'oxygène ou sous une atmosphère contenant de l'eau, et à une température comprise entre 10°C et 50°C, et de préférence à température ambiante. Généralement une durée de maturation inférieure à quarante-huit heures et de préférence comprise entre cinq minutes et cinq heures, est suffisante. Des durées plus longues ne sont pas exclues, mais n'apportent pas nécessairement d'amélioration.

### Etape g) Réduction par un gaz réducteur

Préalablement à l'utilisation du catalyseur dans le réacteur catalytique et la mise en oeuvre d'un procédé d'hydrogénation, on effectue une étape de traitement réducteur g) en présence d'un gaz réducteur de manière à obtenir un catalyseur comprenant du nickel au moins partiellement sous forme métallique. Cette étape est avantageusement réalisée *in-situ* c'est-à-dire après le chargement du catalyseur dans un réacteur d'hydrogénation. Ce traitement permet d'activer ledit catalyseur et de former des particules métalliques, en particulier du nickel à l'état zéro valent. La réalisation *in-situ* du traitement réducteur du catalyseur permet de s'affranchir d'une étape supplémentaire de passivation du catalyseur par un composé oxygéné ou par le CO₂, ce qui est nécessairement le cas lorsque le catalyseur est préparé en réalisant un traitement réducteur ex-situ, c'est-à-dire en dehors du réacteur utilisé pour l'hydrogénation de composés aromatiques ou polyaromatiques. En effet, lorsque le traitement réducteur est réalisé ex-situ, il est nécessaire de réaliser une étape de passivation afin de préserver la phase métallique du catalyseur en présence d'air (lors des opérations de transport et de chargement du catalyseur dans le réacteur d'hydrogénation), puis de réaliser une étape nouvelle étape de réduction du catalyseur.

Le gaz réducteur est de préférence l'hydrogène. L'hydrogène peut être utilisé pur ou en mélange (par exemple un mélange hydrogène/azote, hydrogène/argon, hydrogène/méthane). Dans le cas où l'hydrogène est utilisé en mélange, toutes les proportions sont envisageables.

Selon un aspect essentiel du procédé de préparation selon l'invention, ledit traitement réducteur est réalisé à une température supérieure ou égale à 150°C et inférieure à 250°C, de préférence comprise entre 160 et 230°C, et plus préférentiellement entre 170 et 220°C. La durée du traitement réducteur est comprise entre 5 minutes et moins de 5 heures, de préférence entre 10 minutes et 4 heures, et encore plus préférentiellement entre 10 minutes et 110 minutes.

La présence de l'alliage de nickel-cuivre au moins partiellement sous forme réduite permet de recourir à des conditions opératoires de réduction de la phase active de nickel moins sévères que dans l'art antérieur et permet ainsi de réaliser directement l'étape de réduction au sein du réacteur dans lequel on souhaite réaliser l'hydrogénation de composés insaturés aromatiques.

Par ailleurs, la présence de cuivre dans le catalyseur permet de conserver une bonne activité du catalyseur et une bonne durée de vie du catalyseur lorsque ce dernier est mis en contact avec une charge hydrocarbonée comprenant du soufre. En effet, par rapport au nickel, le cuivre présent dans le catalyseur capte plus facilement les composés soufrés compris dans la charge, ce qui limite l'empoisonnement irréversible des sites actifs. La montée en température jusqu'à la température de réduction désirée est généralement lente, par exemple fixée entre 0,1 et 10°C/min, de préférence entre 0,3 et 7°C/min.

Le débit d'hydrogène, exprimé en L/heure/gramme de précurseur de catalyseur est compris entre 0,01 et 100 L/heure/gramme de catalyseur, de préférence entre 0,05 et 10 L/heure/gramme de précurseur de catalyseur, de façon encore plus préférée entre 0,1 et 5 L/heure/gramme de précurseur de catalyseur.

### Etape h) Passivation (optionnelle)

Le catalyseur préparé selon le procédé selon l'invention peut avantageusement subir une étape de passivation par un composé soufré qui permet d'améliorer la sélectivité des catalyseurs et d'éviter les emballements thermiques lors des démarrages de catalyseurs neufs *(*« *run-away* » selon la terminologie anglo-saxonne). La passivation consiste généralement à empoisonner irréversiblement par le composé soufré les sites actifs les plus virulents du nickel qui existent sur le catalyseur neuf et donc à atténuer l'activité du catalyseur en faveur de sa sélectivité. L'étape de passivation est réalisée par la mise en oeuvre de méthodes connues de l'Homme du métier

L'étape de passivation par un composé soufré est généralement effectuée à une température comprise entre 20 et 350°C, de préférence entre 40 et 200°C, pendant 10 à 240 minutes. Le composé soufré est par exemple choisi parmi les composés suivants: thiophène, thiophane, alkylmonosulfures tels que diméthylsulfure, diéthylsulfure, dipropylsulfure et propylméthylsulfure ou encore un disulfure organique de formule HO-R₁-S-S-R₂-OH tel que le di-thio-di-éthanol de formule HO-C₂H₄-S-S-C₂H₄-OH (appelé souvent DEODS). La teneur en soufre est généralement comprise entre 0,1 et 2 % poids dudit élément par rapport au poids total du catalyseur.

Dans un mode de réalisation selon l'invention, la préparation du catalyseur est effectuée ex-situ, c'est-à-dire avant chargement du catalyseur dans l'unité réactionnelle du procédé d'hydrogénation sélective ou d'hydrogénation des aromatiques.

### Procédé d'hydrogénation sélective

La présente invention a également pour objet un procédé d'hydrogénation sélective de composés polyinsaturés contenant au moins 2 atomes de carbone par molécule, tels que les dioléfines et/ou les acétyléniques et/ou les alcénylaromatiques, aussi appelés styréniques, contenus dans une charge d'hydrocarbures ayant un point d'ébullition final inférieur ou égal à 300°C, lequel procédé étant réalisé à une température comprise entre 0 et 300°C, à une pression comprise entre 0,1 et 10 MPa, à un ratio molaire hydrogène/(composés polyinsaturés à hydrogéner) compris entre 0,1 et 10 et à une vitesse volumique horaire comprise entre 0,1 et 200 h⁻¹ lorsque le procédé est réalisé en phase liquide, ou à un ratio molaire hydrogène/(composés polyinsaturés à hydrogéner) compris entre 0,5 et 1000 et à une vitesse volumique horaire entre 100 et 40000 h⁻¹ lorsque le procédé est réalisé en phase gazeuse, en présence d'un catalyseur obtenu par le procédé de préparation tel que décrit ci-avant dans la description.

Les composés organiques mono-insaturés tels que par exemple l'éthylène et le propylène, sont à la source de la fabrication de polymères, de matières plastiques et d'autres produits chimiques à valeur ajoutée. Ces composés sont obtenus à partir du gaz naturel, du naphta ou du gazole qui ont été traités par des procédés de vapocraquage ou de craquage catalytique. Ces procédés sont opérés à haute température et produisent, en plus des composés mono-insaturés recherchés, des composés organiques polyinsaturés tels que l'acétylène, le propadiène et le méthylacétylène (ou propyne), le 1-2-butadiène et le 1-3-butadiène, le vinylacétylène et l'éthylacétylène, et d'autres composés polyinsaturés dont le point d'ébullition correspond à la coupe C5+ (composés hydrocarbonés ayant au moins 5 atomes de carbone), en particulier des composés dioléfiniques ou styréniques ou indéniques. Ces composés polyinsaturés sont très réactifs et conduisent à des réactions parasites dans les unités de polymérisation. Il est donc nécessaire de les éliminer avant de valoriser ces coupes.

L'hydrogénation sélective est le principal traitement développé pour éliminer spécifiquement les composés polyinsaturés indésirables de ces charges d'hydrocarbures. Elle permet la conversion des composés polyinsaturés vers les alcènes ou aromatiques correspondants en évitant leur saturation totale et donc la formation des alcanes ou naphtènes correspondants. Dans le cas d'essences de vapocraquage utilisées comme charge, l'hydrogénation sélective permet également d'hydrogéner sélectivement les alcénylaromatiques en aromatiques en évitant l'hydrogénation des noyaux aromatiques.

La charge d'hydrocarbures traitée dans le procédé d'hydrogénation sélective a un point d'ébullition final inférieur ou égal à 300°C et contient au moins 2 atomes de carbone par molécule et comprend au moins un composé polyinsaturé. On entend par « composés polyinsaturés » des composés comportant au moins une fonction acétylénique et/ou au moins une fonction diénique et/ou au moins une fonction alcénylaromatique.

Plus particulièrement, la charge est sélectionnée dans le groupe constitué par une coupe C2 de vapocraquage, une coupe C2-C3 de vapocraquage, une coupe C3 de vapocraquage, une coupe C4 de vapocraquage, une coupe C5 de vapocraquage et une essence de vapocraquage encore appelée essence de pyrolyse ou coupe C5+.

La coupe C2 de vapocraquage, avantageusement utilisée pour la mise en oeuvre du procédé d'hydrogénation sélective selon l'invention, présente par exemple la composition suivante : entre 40 et 95 % poids d'éthylène, de l'ordre de 0,1 à 5 % poids d'acétylène, le reste étant essentiellement de l'éthane et du méthane. Dans certaines coupes C2 de vapocraquage, entre 0,1 et 1 % poids de composés en C3 peut aussi être présent.

La coupe C3 de vapocraquage, avantageusement utilisée pour la mise en oeuvre du procédé d'hydrogénation sélective selon l'invention, présente par exemple la composition moyenne suivante : de l'ordre de 90 % poids de propylène, de l'ordre de 1 à 8 % poids de propadiène et de méthylacétylène, le reste étant essentiellement du propane. Dans certaines coupes C3, entre 0,1 et 2 % poids de composés en C2 et de composés en C4 peut aussi être présent.

Une coupe C2 - C3 peut aussi être avantageusement utilisée pour la mise en oeuvre du procédé d'hydrogénation sélective selon l'invention. Elle présente par exemple la composition suivante : de l'ordre de 0,1 à 5 % poids d'acétylène, de l'ordre de 0,1 à 3 % poids de propadiène et de méthylacétylène, de l'ordre de 30 % poids d'éthylène, de l'ordre de 5 % poids de propylène, le reste étant essentiellement du méthane, de l'éthane et du propane. Cette charge peut aussi contenir entre 0,1 et 2 % poids de composés en C4.

La coupe C4 de vapocraquage, avantageusement utilisée pour la mise en oeuvre du procédé d'hydrogénation sélective selon l'invention, présente par exemple la composition massique moyenne suivante : 1 % poids de butane, 46,5 % poids de butène, 51 % poids de butadiène, 1,3 % poids de vinylacétylène et 0,2 % poids de butyne. Dans certaines coupes C4, entre 0,1 et 2 % poids de composés en C3 et de composés en C5 peut aussi être présent.

La coupe C5 de vapocraquage, avantageusement utilisée pour la mise en oeuvre du procédé d'hydrogénation sélective selon l'invention, présente par exemple la composition suivante : 21 % poids de pentanes, 45 % poids de pentènes, 34 % poids de pentadiènes.

L'essence de vapocraquage ou essence de pyrolyse, avantageusement utilisée pour la mise en oeuvre du procédé d'hydrogénation sélective selon l'invention, correspond à une coupe hydrocarbonée dont la température d'ébullition est généralement comprise entre 0 et 300°C, de préférence entre 10 et 250°C. Les hydrocarbures polyinsaturés à hydrogéner présents dans ladite essence de vapocraquage sont en particulier des composés dioléfiniques (butadiène, isoprène, cyclopentadiène...), des composés styréniques (styrène, alpha-méthylstyrène...) et des composés indéniques (indène...). L'essence de vapocraquage comprend généralement la coupe C5-C12 avec des traces de C3, C4, C13, C14, C15 (par exemple entre 0,1 et 3% poids pour chacune de ces coupes). Par exemple, une charge formée d'essence de pyrolyse a généralement une composition suivante: 5 à 30 % poids de composés saturés (paraffines et naphtènes), 40 à 80 % poids de composés aromatiques, 5 à 20 % poids de mono-oléfines, 5 à 40 % poids de dioléfines, 1 à 20 % poids de composés alcénylaromatiques, l'ensemble des composés formant 100 %. Elle contient également de 0 à 1000 ppm poids de soufre, de préférence de 0 à 500 ppm poids de soufre.

De manière préférée, la charge d'hydrocarbures polyinsaturés traitée conformément au procédé d'hydrogénation sélective selon l'invention est une coupe C2 de vapocraquage, ou une coupe C2-C3 de vapocraquage, ou une essence de vapocraquage.

Le procédé d'hydrogénation sélective selon l'invention vise à éliminer lesdits hydrocarbures polyinsaturés présents dans ladite charge à hydrogéner sans hydrogéner les hydrocarbures monoinsaturés. Par exemple, lorsque ladite charge est une coupe C2, le procédé d'hydrogénation sélective vise à hydrogéner sélectivement l'acétylène. Lorsque ladite charge est une coupe C3, le procédé d'hydrogénation sélective vise à hydrogéner sélectivement le propadiène et le méthylacétylène. Dans le cas d'une coupe C4, on vise à éliminer le butadiène, le vinylacétylène (VAC) et le butyne, dans le cas d'une coupe C5, on vise à éliminer les pentadiènes. Lorsque ladite charge est une essence de vapocraquage, le procédé d'hydrogénation sélective vise à hydrogéner sélectivement lesdits hydrocarbures polyinsaturés présents dans ladite charge à traiter de manière à ce que les composés dioléfiniques soient partiellement hydrogénés en mono-oléfines et que les composés styréniques et indéniques soient partiellement hydrogénés en composés aromatiques correspondants en évitant l'hydrogénation des noyaux aromatiques.

La mise en oeuvre technologique du procédé d'hydrogénation sélective est par exemple réalisée par injection, en courant ascendant ou descendant, de la charge d'hydrocarbures polyinsaturés et de l'hydrogène dans au moins un réacteur à lit fixe. Ledit réacteur peut être de type isotherme ou de type adiabatique. Un réacteur adiabatique est préféré. La charge d'hydrocarbures polyinsaturés peut avantageusement être diluée par une ou plusieurs ré-injection(s) de l'effluent, issu dudit réacteur où se produit la réaction d'hydrogénation sélective, en divers points du réacteur, situés entre l'entrée et la sortie du réacteur afin de limiter le gradient de température dans le réacteur. La mise en oeuvre technologique du procédé d'hydrogénation sélective selon l'invention peut également être avantageusement réalisée par l'implantation d'au moins dudit catalyseur supporté dans une colonne de distillation réactive ou dans des réacteurs - échangeurs ou dans un réacteur de type slurry. Le flux d'hydrogène peut être introduit en même temps que la charge à hydrogéner et/ou en un ou plusieurs points différents du réacteur.

L'hydrogénation sélective des coupes C2, C2-C3, C3, C4, C5 et C5+ de vapocraquage peut être réalisée en phase gazeuse ou en phase liquide, de préférence en phase liquide pour les coupes C3, C4, C5 et C5+ et en phase gazeuse pour les coupes C2 et C2-C3. Une réaction en phase liquide permet d'abaisser le coût énergétique et d'augmenter la durée de cycle du catalyseur.

D'une manière générale, l'hydrogénation sélective d'une charge d'hydrocarbures contenant des composés polyinsaturés contenant au moins 2 atomes de carbone par molécule et ayant un point d'ébullition final inférieur ou égal à 300°C s'effectue à une température comprise entre 0 et 300°C, à une pression comprise entre 0,1 et 10 MPa, à un ratio molaire hydrogène/(composés polyinsaturés à hydrogéner) compris entre 0,1 et 10 et à une vitesse volumique horaire V.V.H. (définie comme le rapport du débit volumique de charge sur le volume du catalyseur) comprise entre 0,1 et 200 h⁻¹ pour un procédé réalisé en phase liquide, ou à un ratio molaire hydrogène/(composés polyinsaturés à hydrogéner) compris entre 0,5 et 1000 et à une vitesse volumique horaire V.V.H. comprise entre 100 et 40000 h⁻¹ pour un procédé réalisé en phase gazeuse.

Dans un mode de réalisation selon l'invention, lorsqu'on effectue un procédé d'hydrogénation sélective dans lequel la charge est une essence de vapocraquage comportant des composés polyinsaturés, le ratio molaire (hydrogène)/(composés polyinsaturés à hydrogéner) est généralement compris entre 0,5 et 10, de préférence entre 0,7 et 5,0 et de manière encore plus préférée entre 1,0 et 2,0, la température est comprise entre 0 et 200°C, de préférence entre 20 et 200 °C et de manière encore plus préférée entre 30 et 180°C, la vitesse volumique horaire (V.V.H.) est comprise généralement entre 0,5 et 100 h⁻¹, de préférence entre 1 et 50 h⁻¹ et la pression est généralement comprise entre 0,3 et 8,0 MPa, de préférence entre 1,0 et 7,0 MPa et de manière encore plus préférée entre 1,5 et 4,0 MPa.

Plus préférentiellement, on effectue un procédé d'hydrogénation sélective dans lequel la charge est une essence de vapocraquage comportant des composés polyinsaturés, le ratio molaire hydrogène/(composés polyinsaturés à hydrogéner) est compris entre 0,7 et 5,0, la température est comprise entre 20 et 200 °C, la vitesse volumique horaire (V.V.H.) est comprise généralement entre 1 et 50 h⁻¹ et la pression est comprise entre 1,0 et 7,0 MPa.

Encore plus préférentiellement, on effectue un procédé d'hydrogénation sélective dans lequel la charge est une essence de vapocraquage comportant des composés polyinsaturés, le ratio molaire hydrogène/(composés polyinsaturés à hydrogéner) est compris entre 1,0 et 2,0, la température est comprise entre 30 et 180°C, la vitesse volumique horaire (V.V.H.) est comprise généralement entre 1 et 50 h⁻¹ et la pression est comprise entre 1,5 et 4,0 MPa.

Le débit d'hydrogène est ajusté afin d'en disposer en quantité suffisante pour hydrogéner théoriquement l'ensemble des composés polyinsaturés et de maintenir un excès d'hydrogène en sortie de réacteur.

Dans un autre mode de réalisation selon l'invention, lorsqu'on effectue un procédé d'hydrogénation sélective dans lequel la charge est une coupe C2 de vapocraquage et/ou une coupe C2-C3 de vapocraquage comportant des composés polyinsaturés, le ratio molaire (hydrogène)/(composés polyinsaturés à hydrogéner) est généralement compris entre 0,5 et 1000, de préférence entre 0,7 et 800, la température est comprise entre 0 et 300°C, de préférence entre 15 et 280 °C, la vitesse volumique horaire (V.V.H.) est comprise généralement entre 100 et 40000 h⁻¹, de préférence entre 500 et 30000 h⁻¹ et la pression est généralement comprise entre 0,1 et 6,0 MPa, de préférence entre 0,2 et 5,0 MPa.

### Procédé d'hydrogénation des aromatiques

La présente invention a également pour objet un procédé d'hydrogénation d'au moins un composé aromatique ou polyaromatique contenu dans une charge d'hydrocarbures ayant un point d'ébullition final inférieur ou égal à 650°C, généralement entre 20 et 650°C, et de préférence entre 20 et 450°C. Ladite charge d'hydrocarbures contenant au moins un composé aromatique ou polyaromatique peut être choisi parmi les coupes pétrolières ou pétrochimiques suivantes : le reformat du reformage catalytique, le kérosène, le gazole léger, le gazole lourd, les distillats de craquage, tels que l'huile de recyclage de FCC, le gazole d'unité de cokéfaction, les distillats d'hydrocraquage.

La teneur en composés aromatiques ou polyaromatiques contenus dans la charge d'hydrocarbures traitée dans le procédé d'hydrogénation selon l'invention est généralement compris entre 0,1 et 80% en poids, de préférence entre 1 et 50% en poids, et de manière particulièrement préférée entre 2 et 35% en poids, le pourcentage étant basé sur le poids total de la charge d'hydrocarbures. Les composés aromatiques présents dans ladite charge d'hydrocarbures sont par exemple le benzène ou des alkylaromatiques tels que le toluène, l'éthylbenzène, l'o-xylène, le m-xylène, ou le p-xylène, ou encore des aromatiques ayant plusieurs noyaux aromatiques (polyaromatiques) tels que le naphtalène.

La teneur en soufre ou en chlore de la charge est généralement inférieure à 5000 ppm poids de soufre ou de chlore, de préférence inférieure à 100 ppm poids, et de manière particulièrement préférée inférieure à 10 ppm poids.

La mise en oeuvre technologique du procédé d'hydrogénation des composés aromatiques ou polyaromatiques est par exemple réalisée par injection, en courant ascendant ou descendant, de la charge d'hydrocarbures et de l'hydrogène dans au moins un réacteur à lit fixe. Ledit réacteur peut être de type isotherme ou de type adiabatique. Un réacteur adiabatique est préféré. La charge d'hydrocarbures peut avantageusement être diluée par une ou plusieurs ré-injection(s) de l'effluent, issu dudit réacteur où se produit la réaction d'hydrogénation des aromatiques, en divers points du réacteur, situés entre l'entrée et la sortie du réacteur afin de limiter le gradient de température dans le réacteur. La mise en oeuvre technologique du procédé d'hydrogénation des aromatiques selon l'invention peut également être avantageusement réalisée par l'implantation d'au moins dudit catalyseur supporté dans une colonne de distillation réactive ou dans des réacteurs - échangeurs ou dans un réacteur de type slurry. Le flux d'hydrogène peut être introduit en même temps que la charge à hydrogéner et/ou en un ou plusieurs points différents du réacteur.

L'hydrogénation des composés aromatiques ou polyaromatiques peut être réalisée en phase gazeuse ou en phase liquide, de préférence en phase liquide. D'une manière générale, l'hydrogénation des composés aromatiques ou polyaromatiques s'effectue à une température comprise entre 30 et 350°C, de préférence entre 50 et 325°C, à une pression comprise entre 0,1 et 20 MPa, de préférence entre 0,5 et 10 MPa, à un ratio molaire hydrogène/(composés aromatiques à hydrogéner) entre 0,1 et 10 et à une vitesse volumique horaire V.V.H. comprise entre 0,05 et 50 h⁻¹, de préférence entre 0,1 et 10 h⁻¹ d'une charge d'hydrocarbures contenant des composés aromatiques ou polyaromatiques et ayant un point d'ébullition final inférieur ou égal à 650°C, généralement entre 20 et 650°C, et de préférence entre 20 et 450°C.

Le débit d'hydrogène est ajusté afin d'en disposer en quantité suffisante pour hydrogéner théoriquement l'ensemble des composés aromatiques et de maintenir un excès d'hydrogène en sortie de réacteur.

La conversion des composés aromatiques ou polyaromatiques est généralement supérieure à 20% en mole, de préférence supérieure à 40% en mole, de manière plus préférée supérieure à 80% en mole, et de manière particulièrement préférée supérieure à 90 % en mole des composés aromatiques ou polyaromatiques contenus dans la charge hydrocarbonée. La conversion se calcule en divisant la différence entre les moles totales des composés aromatiques ou polyaromatiques dans la charge d'hydrocarbures et dans le produit par les moles totales des composés aromatiques ou polyaromatiques dans la charge d'hydrocarbures.

Selon une variante particulière du procédé selon l'invention, on réalise un procédé d'hydrogénation du benzène d'une charge d'hydrocarbures, tel que le reformat issu d'une unité de reformage catalytique. La teneur en benzène dans ladite charge d'hydrocarbures est généralement comprise entre 0,1 et 40% poids, de préférence entre 0,5 et 35% poids, et de manière particulièrement préférée entre 2 et 30% poids, le pourcentage en poids étant basé sur le poids total de la charge d'hydrocarbures.

La teneur en soufre ou en chlore de la charge est généralement inférieure à 10 ppm poids de soufre ou chlore respectivement, et de préférence inférieure à 2 ppm poids.

L'hydrogénation du benzène contenu dans la charge d'hydrocarbures peut être réalisée en phase gazeuse ou en phase liquide, de préférence en phase liquide. Lorsqu'elle est réalisée en phase liquide, un solvant peut être présent, tel que le cyclohexane, l'heptane, l'octane. D'une manière générale, l'hydrogénation du benzène s'effectue à une température comprise entre 30 et 250°C, de préférence entre 50 et 200°C, et de manière plus préférée entre 80 et 180°C, à une pression comprise entre 0,1 et 10 MPa, de préférence entre 0,5 et 4 MPa, à un ratio molaire hydrogène/(benzène) entre 0,1 et 10 et à une vitesse volumique horaire V.V.H. comprise entre 0,05 et 50 h⁻¹, de préférence entre 0,5 et 10 h⁻¹.

La conversion du benzène est généralement supérieure à 50% en mole, de préférence supérieure à 80% en mole, de manière plus préférée supérieure à 90% en mole et de manière particulièrement préférée supérieure à 98 % en mole.

L'invention va maintenant être illustré via les exemples ci-après qui ne sont nullement limitatifs.

### Exemples

### Exemple 1 : Préparation de l'alumine AL-1

Un gel d'alumine est synthétisé via un mélange d'aluminate de sodium et de sulfate d'aluminium. La réaction de précipitation se fait à une température de 60°C, à un pH de 9, durant 60 minutes et sous une agitation de 200 tr/min.

Le gel ainsi obtenu subit un malaxage sur un malaxeur bras en Z pour fournir la pâte. L'extrusion est réalisée par passage de la pâte à travers une filière munie d'orifices de diamètre 1,6 mm en forme de trilobe. Les extrudés ainsi obtenus sont séchés sous flux d'air sec à 150°C pendant 12 heures puis calciné à 450°C sous flux d'air sec pendant 5 heures. L'extrudé subit un traitement hydrothermal à 650°C en présence d'une solution aqueuse contenant de l'acide acétique à 6,5% poids par rapport au poids d"alumine pendant 3 heures en autoclave, puis est calciné sous air sec à 1000°C pendant 2 heures en réacteur tubulaire. On obtient l'alumine AL-1.

L'alumine AL-1 présente une surface spécifique de 80 m²/g, un volume poreux (déterminé par porosimétrie au Hg) de 0,85 mL/g et un diamètre mésoporeux de 35 nm.

La teneur en sodium est de 0,0350% en poids par rapport au poids total de l'alumine et la teneur en soufre est de 0,15% en poids par rapport au poids total de l'alumine.

### Exemple 1bis : Préparation de l'alumine AL-2

Un gel d'alumine est synthétisé via un mélange d'aluminate de sodium et de sulfate d'aluminium. La réaction de précipitation se fait à une température de 60°C, à un pH de 9, durant 60 minutes et sous une agitation de 200 tr/min.

Le gel ainsi obtenu subit un malaxage sur un malaxeur bras en Z pour fournir la pâte. L'extrusion est réalisée par passage de la pâte à travers une filière munie d'orifices de diamètre 1,6 mm en forme de trilobe. Les extrudés ainsi obtenus sont séchés sous flux d'air sec à 150°C pendant 12 heures puis calciné à 450°C sous flux d'air sec pendant 5 heures. On obtient l'alumine AL-2.

L'alumine AL-2 présente une surface spécifique de 255 m²/g, un volume poreux (déterminé par porosimétrie au Hg) de 0,7 mL/g et un diamètre mésoporeux de 12 nm.

La teneur en sodium est de 0,0350% en poids par rapport au poids total de l'alumine et la teneur en soufre est de 0,15% en poids par rapport au poids total de l'alumine.

### Exemple 2 : Préparation d'une solution aqueuse de précurseurs de Ni

La solution aqueuse de précurseurs de Ni (solution S1) utilisée pour la préparation du catalyseur A est préparée en dissolvant 43,5 g de nitrate de nickel (NiNOs, fournisseur Stream Chemicals^{®}) dans un volume de 13 mL d'eau distillée. On obtient la solution S1 dont la concentration en Ni est de 350 g de Ni par litre de solution.

### Exemple 3 : Préparation d'une solution aqueuse de précurseurs de Ni avec composé organique (selon l'étape xx)

La solution aqueuse de précurseurs de Ni (solution S2) utilisée pour la préparation des catalyseurs B à G est préparée en dissolvant 43,5 g de nitrate de nickel (NiNO₃, fournisseur Strem Chemicals^{®}) et de l'acide malonique (CAS 141-82-2 ; fournisseur Fluka^{®}) dans un volume de 13 mL d'eau distillée. Le ratio molaire additif/Ni étant de 0,5. On obtient la solution S2 dont la concentration en Ni est de 350 g de Ni par litre de solution.

### Exemple 4 : Préparation d'une solution aqueuse des précurseurs de l'alliage NiCu (5%Ni)

La solution aqueuse de précurseurs de Ni (solution S3) utilisée pour la préparation des catalyseurs C, D, E, et G est préparée en dissolvant 14,5 g de nitrate de nickel (NiNOs, fournisseur Strem Chemicals^{®}) dans un volume de 13 mL d'eau distillée. On obtient une solution dont la concentration en Ni est de 116,6 g de Ni par litre de solution. Le précurseur Nitrate de cuivre est ensuite ajouté afin d'avoir notamment un ratio molaire Ni/Cu de 3 (catalyseurs C à F). On obtient la solution S3. Elle permet d'introduire les précurseurs de l'alliage NiCu avec une teneur massique en Ni par rapport au catalyseur final d'environ 5%pds.

### Exemple 5 : Préparation d'un catalyseur A

La solution S2 préparée à l'exemple 3 est imprégnée à sec, en l'ajoutant goutte-à-goutte, sur 10 g d'alumine AL-1 obtenue selon l'exemple 1.

Le solide ainsi obtenu est ensuite séché en étuve pendant 12 heures à 120°C, puis calciné sous un flux d'air sec de 1 L/h/g de catalyseur à 450°C pendant 2 heures.

L'air sec utilisé dans cet exemple et dans tous les exemples ci-dessous contient moins de 5 grammes d'eau par kilogramme d'air

Le précurseur de catalyseur ainsi obtenu est imprégné à sec avec une solution aqueuse contenant de l'acide formique avec le ratio molaire HCOOH/Ni égal à 1 mol/mol.

A l'issue de l'imprégnation de la solution aqueuse contenant l'acide formique, le précurseur de catalyseur subit un traitement thermique à 150°C, 2 heures sous un flux d'air contenant 50 grammes d'eau par kilogramme d'air sec avec un débit de 1 L/h/g de catalyseur, puis pendant 1 heure à 120°C sous flux d'air sec.

Ensuite la solution S3 est imprégnée à sec sur le précurseur de catalyseur ci -dessus. La teneur en Ni visée sur cette étape est de 5% en poids de Ni par rapport au poids du catalyseur final. Le solide ainsi obtenu est ensuite séché en étuve pendant une nuit à 120°C, puis calciné sous un flux d'air de 1 L/h/g de catalyseur à 450°C pendant 2 heures.

Le précurseur de catalyseur est ensuite réduit dans les conditions telles que décrites à l'exemple 12 ci-après.

On obtient le catalyseur A dont les caractéristiques sont reportées dans le tableau 1 et 2 ci-dessous.

### Exemple 6 : Préparation d'un catalyseur B selon invention

La solution S3 est imprégnée à sec goutte à goutte sur 10 g du support AL-1. La teneur en Ni visée sur cette étape est de 5% en poids de Ni par rapport au poids du catalyseur final. Le solide ainsi obtenu est ensuite séché en étuve pendant une nuit à 120°C, puis calciné sous un flux d'air de 1 L/h/g de catalyseur à 450°C pendant 2 heures. On obtient le précurseur du catalyseur final, B'.

Ensuite, la solution S2 préparée à l'exemple 3 est imprégnée à sec, en l'ajoutant goutte-à-goutte, le précurseur de catalyseur final B'.

Le solide ainsi obtenu est ensuite séché en étuve pendant 12 heures à 120°C, puis calciné sous un flux d'air sec de 1 L/h/g de catalyseur à 450°C pendant 2 heures.

Le précurseur de catalyseur obtenu est imprégné à sec avec une solution aqueuse contenant de l'acide formique avec le ratio molaire HCOOH/Ni égal à 1 mol/mol.

A l'issue de l'imprégnation de la solution aqueuse contenant l'acide formique, le précurseur de catalyseur subit un traitement thermique à 150°C, 2 heures sous un flux d'air contenant 50 grammes d'eau par kilogramme d'air sec avec un débit de 1 L/h/g de catalyseur, puis pendant 1 heure à 120°C sous flux d'air sec. Le précurseur de catalyseur est ensuite réduit dans les conditions telles que décrites à l'exemple 12 ci-après.

On obtient le catalyseur B dont les caractéristiques sont reportées dans le tableau 1 et 2 ci-dessous.

### Exemple 7 : Préparation d'un catalyseur C (non-conforme)

La solution S3 est imprégnée à sec goutte à goutte sur 10g du support AL-1. La teneur en Ni visée sur cette étape est de 5% en poids de Ni par rapport au poids du catalyseur final. Le solide ainsi obtenu est ensuite séché en étuve pendant une nuit à 120°C, puis calciné sous un flux d'air de 1 L/h/g de catalyseur à 450°C pendant 2 heures.

On obtient le précurseur du catalyseur final, C'.

La solution S1 préparée à l'exemple 2 est ensuite imprégnée à sec, en l'ajoutant goutte-à-goutte, le précurseur du catalyseur C'.

Le solide ainsi obtenu est ensuite séché en étuve pendant 12 heures à 120°C, puis calciné sous un flux d'air sec de 1 L/h/g de catalyseur à 450°C pendant 2 heures.

Le précurseur de catalyseur ainsi obtenu est imprégné à sec avec une solution aqueuse contenant de l'acide formique avec le ratio molaire HCOOH/Ni égal à 1 mol/mol.

A l'issue de l'imprégnation de la solution aqueuse contenant l'acide formique, le précurseur de catalyseur subit un traitement thermique à 150°C, 2 heures sous un flux d'air contenant 50 grammes d'eau par kilogramme d'air sec avec un débit de 1 L/h/g de catalyseur, puis pendant 1 heure à 120°C sous flux d'air sec. Le précurseur de catalyseur est ensuite réduit dans les conditions telles que décrites à l'exemple 12 ci-après.

On obtient le catalyseur C dont les caractéristiques sont reportées dans le tableau 1 et 2 ci-dessous.

### Exemple 8: Préparation d'un catalyseur D (non-conforme)

La solution S3 est imprégnée à sec goutte à goutte sur 10g du support AL-2. La teneur en Ni visée sur cette étape est de 5% en poids de Ni par rapport au poids du catalyseur final. Le solide ainsi obtenu est ensuite séché en étuve pendant une nuit à 120°C, puis calciné sous un flux d'air de 1 L/h/g de catalyseur à 450°C pendant 2 heures.

On obtient le précurseur du catalyseur final, D'.

La solution S2 préparée à l'exemple 3 est ensuite imprégnée à sec, en l'ajoutant goutte-à-goutte, sur le précurseur du catalyseur final, D'.

Le solide ainsi obtenu est ensuite séché en étuve pendant 12 heures à 120°C, puis calciné sous un flux d'air sec de 1 L/h/g de catalyseur à 450°C pendant 2 heures.

Le précurseur de catalyseur ainsi obtenu est imprégné à sec avec une solution aqueuse contenant de l'acide formique avec le ratio molaire HCOOH/Ni égal à 1 mol/mol.

A l'issue de l'imprégnation de la solution aqueuse contenant l'acide formique, le précurseur de catalyseur subit un traitement thermique à 150°C, 2 heures sous un flux d'air contenant 50 grammes d'eau par kilogramme d'air sec avec un débit de 1 L/h/g de catalyseur, puis pendant 1 heure à 120°C sous flux d'air sec. Le précurseur de catalyseur est ensuite réduit dans les conditions telles que décrites à l'exemple 12 ci-après.

On obtient le catalyseur D dont les caractéristiques sont reportées dans le tableau 1 et 2 ci-dessous.

### Exemple 9 : Préparation d'un catalyseur E (non-conforme)

La solution S2 préparée à l'exemple 3 est imprégnée à sec, en l'ajoutant goutte-à-goutte, sur 10 g d'alumine AL-1 obtenue selon l'exemple 1.

Le solide ainsi obtenu est ensuite séché en étuve pendant 12 heures à 120°C, puis calciné sous un flux d'air sec de 1 L/h/g de catalyseur à 450°C pendant 2 heures.

L'air sec utilisé dans cet exemple et dans tous les exemples ci-dessous contient moins de 5 grammes d'eau par kilogramme d'air

Le précurseur de catalyseur E' ainsi obtenu est imprégné à sec avec une solution aqueuse contenant de l'acide formique avec le ratio molaire HCOOH/Ni égal à 1 mol/mol.

A l'issue de l'imprégnation de la solution aqueuse contenant l'acide formique, le précurseur de catalyseur subit un traitement thermique à 150°C, 2 heures sous un flux d'air contenant 50 grammes d'eau par kilogramme d'air sec avec un débit de 1 L/h/g de catalyseur, puis pendant 1 heure à 120°C sous flux d'air sec.

On obtient le catalyseur E dont les caractéristiques sont reportées dans le tableau 1 et 2 ci-dessous.

Le précurseur de catalyseur est ensuite réduit dans les conditions telles que décrites à l'exemple 12 ci-après.

### Exemple 10 : Préparation d'un catalyseur F (non-conforme)

La solution S3 est imprégnée à sec goutte à goutte sur 10g du support AL-1. La teneur en Ni visée sur cette étape est de 5% en poids de Ni par rapport au poids du catalyseur final. Le solide ainsi obtenu est ensuite séché en étuve pendant une nuit à 120°C, puis calciné sous un flux d'air de 1 L/h/g de catalyseur à 450°C pendant 2 heures.

On obtient le précurseur du catalyseur final, F'.

La solution S2 préparée à l'exemple 3 est imprégnée à sec, en l'ajoutant goutte-à-goutte, sur le précurseur de catalyseur F'.

Le solide ainsi obtenu est ensuite séché en étuve pendant 12 heures à 120°C, puis calciné sous un flux d'air sec de 1 L/h/g de catalyseur à 450°C pendant 2 heures.

On obtient le catalyseur F dont les caractéristiques sont reportées dans le tableau 1 et 2 ci-dessous ci-après.

Le précurseur de catalyseur est ensuite réduit dans les conditions telles que décrites à l'exemple 12 ci-après.

### Exemple 11 : Préparation d'un catalyseur G (non-conforme)

La solution S2 préparée à l'exemple 3 est imprégnée à sec, en l'ajoutant goutte-à-goutte, sur 10 g d'alumine AL-1 obtenue selon l'exemple 1.

Le solide ainsi obtenu est ensuite séché en étuve pendant 12 heures à 120°C, puis calciné sous un flux d'air sec de 1 L/h/g de catalyseur à 450°C pendant 2 heures. Le précurseur de catalyseur est ensuite réduit dans les conditions telles que décrites à l'exemple 9 ci-après. On obtient le catalyseur G dont les caractéristiques sont reportées dans le tableau 1 et 2 ci-dessous ci-après.

Le précurseur de catalyseur est ensuite réduit dans les conditions telles que décrites à l'exemple 12 ci-après.

### Exemple 12 : Caractérisation

Tous les catalyseurs contiennent les teneurs visées lors de l'imprégnation c'est-à-dire 15% en élément nickel (caractérisé par Fluorescence X) par rapport au poids total du catalyseur, et le % de Cuivre ajouté (caractérisé par Fluorescence X).

La quantité d'alliage obtenue après l'étape de calcination puis réduction a été déterminée par analyse par diffraction des rayons X (DRX) sur des échantillons de catalyseur sous forme de poudre.

La quantité de nickel sous forme métallique obtenue après l'étape de réduction a été déterminée par analyse par diffraction des rayons X (DRX) sur des échantillons de catalyseur sous forme de poudre. Entre l'étape de réduction et pendant toute la durée de la caractérisation par DRX les catalyseurs ne sont jamais remis à l'air libre. Les diagrammes de diffraction sont obtenus par analyse radiocristallographique au moyen d'un diffractomètre en utilisant la méthode classique des poudres avec le rayonnement Kα1 du cuivre (λ = 1,5406 Å).

Le taux de réduction a été calculé en calculant l'aire de la raie de Ni⁰ située vers 52°2θ, sur l'ensemble des diffractogrammes de chaque échantillon de catalyseur analysé, puis en soustrayant le signal présent dès la température ambiante sous la raie à 52° et qui est dû à l'alumine.

La tableau 1 ci-après rassemble les taux de réduction ou encore la teneur en nickel métallique Ni° (exprimée en % poids par rapport au poids total de nickel « actif » ;i.e. sans prendre en compte le nickel qui compose l'alliage) pour tous les catalyseurs A à E caractérisés par DRX après une étape de réduction à 170°C pendant 90 minutes sous flux d'hydrogène. Ces valeurs ont également été comparées avec le taux de réduction obtenu pour le catalyseur A (Ni seul) après une étape de réduction classique (c'est-à-dire à une température de 400°C pendant 15 heures sous flux d'hydrogène).

A température ambiante sur tous les catalyseurs, après calcination, contenant du cuivre et du nickel, nous détectons de l'alumine sous forme delta et thêta, et des grandes raies de NiO et de CuO.

Nous détectons par ailleurs après réduction une raie correspondant à l'alliage sous forme Ni_{0,76}Cu_{0,24}.

Afin d'évaluer le taux de réductibilité et donc la formation du Ni⁰, on mesure l'aire de la raie de Ni⁰ située vers 52°2θ, sur l'ensemble des diffractogrammes, en soustrayant le signal présent dès la température ambiante sous la raie à 52° et qui est dû à l'alumine. On peut ainsi déterminer le pourcentage relatif de Ni⁰ cristallisé après la réduction.

Le tableau 1 ci-dessous récapitule les taux de réductibilité ou encore la teneur en Ni° pour tous les catalyseurs caractérisés par DRX après réduction à 170°C pendant 90 minutes sous flux d'hydrogène. Ces valeurs ont également été comparées avec le taux de réduction obtenu pour le catalyseur G (Ni seul) après une étape de réduction classique (c'est-à-dire à une température de 400°C pendant 15 heures sous flux d'hydrogène).

**Tableau 1**

| Catalyseur | Réduction finale | Teneur Ni pour la 1^{ère} imp. (% pds) | Teneur Ni pour la 2^{ème} imp. (% pds) | Ratio molaire Ni/Cu | Pourcentage de Ni° seul (DRX) après réduction (%) |
|---|---|---|---|---|---|
| A (invention) | 170°C, 90 min | 15 | 5 | 3 | 90 |
| B (invention) | 170°C, 90 min | 5 | 15 | 3 | 95 |
| C (comparatif) | 170°C, 90 min | 5 | 15 | 3 | 100 |
| D (comparatif) | 170°C, 90 min | 5 | 15 | 3 | 100 |
| E (comparatif) | 170°C, 90 min | - | 15 | - | 0 |
| F (comparatif) | 170°C, 90 min | 5 | 15 | | 95 |
| G (comparatif) | 170°C, 90 min | 15 | - | | 70 |
| G (comparatif) | 400°C, 15h | 15 | - | - | 0 |

**Tableau 2**

| Catalyseur | Support | Ajout NiCu | Taille des particules (nm)* | Epaisseur de croute/ diamètre grain (%) | Ratio de densité en Ni entre la croûte et le coeur (dCroute/ d)coeur | Teneur en Ni dans la croûte /Ni total (%) |
|---|---|---|---|---|---|---|
| A | AL-1 | Post-imprégnation | 2,2 | 6,8 | 5 | 66 |
| B | AL-1 | Pré-imprégnation | 2,1 | 5 | 11 | 72 |
| C (non conforme) | AL-1 | Pré-imprégnation | 14 | 4,2 | 6 | 65 |
| D (non conforme) | AL-2 | Pré-imprégnation | 10,2 | Répartition homogène | - | - |
| E (non conforme) | AL-1 | - | 3 | 7 | 8 | 68 |
| F (non conforme) | AL-1 | Pré imprégnation | 2,5 | Répartition homogène | - | - |
| G (non conforme) | AL-1 | - | 2,4 | Répartition homogène | - | - |

| | | | | | | |
|---|---|---|---|---|---|---|
| *Taille des particules des 15% du Nickel qui ne composent pas l'alliage. | | | | | | |

### Exemple 13 : Tests catalytiques : performances en hydrogénation sélective d'un mélange contenant du styrène et de l'isoprène (A_{HYD1})

Les catalyseurs A à G décrits dans les exemples ci-dessus sont testés vis-à-vis de la réaction d'hydrogénation sélective d'un mélange contenant du styrène et de l'isoprène.

La composition de la charge à hydrogéner sélectivement est la suivante : 8 % pds styrène (fournisseur Sigma Aldrich^{®}, pureté 99%), 8 % pds isoprène (fournisseur Sigma Aldrich^{®}, pureté 99%), 84 % pds n-heptane (solvant) (fournisseur VWR^{®}, pureté > 99% chromanorm HPLC). Cette composition correspond à la composition initiale du mélange réactionnel. Ce mélange de molécules modèles est représentatif d'une essence de pyrolyse.

La réaction d'hydrogénation sélective est opérée dans un autoclave de 500 mL en acier inoxydable, muni d'une agitation mécanique à entraînement magnétique et pouvant fonctionner sous une pression maximale de 100 bar (10 MPa) et des températures comprises entre 5°C et 200°C.

Dans un autoclave sont ajoutés 214 mL de n-heptane (fournisseur VWR^{®}, pureté > 99% chromanorm HPLC) et une quantité de 3 mL de catalyseur. L'autoclave est fermé et purgé. Ensuite l'autoclave est pressurisé sous 35 bar (3,5 MPa) d'hydrogène. Le catalyseur est d'abord réduit *in situ,* à 170 °C pendant 90 minutes sous un flux d'hydrogène de 1 L/h/g (rampe de montée en température de 1 °C/min) pour les catalyseurs A à G (ce qui correspond ici à l'étape g) du procédé de préparation selon l'invention selon un mode de réalisation). Ensuite l'autoclave est porté à la température du test égale à 30°C. Au temps t=0, environ 30 g d'un mélange contenant du styrène, de l'isoprène et du du n-heptane sont introduits dans l'autoclave. Le mélange réactionnel a alors la composition décrite ci-dessus et l'agitation est mise en route à 1600 tr/min. La pression est maintenue constante à 35 bar (3,5 MPa) dans l'autoclave à l'aide d'une bouteille réservoir située en amont du réacteur.

Un autre test a été effectué pour le catalyseur A, mais avec une température de réduction du catalyseur de 400°C pendant 15 heures.

L'avancement de la réaction est suivi par prélèvement d'échantillons du milieu réactionnel à intervalles de temps réguliers : le styrène est hydrogéné en éthylbenzène, sans hydrogénation du cycle aromatique, et l'isoprène est hydrogéné en méthyl-butènes. Si la réaction est prolongée plus longtemps que nécessaire, les méthyl-butènes sont à leur tour hydrogénés en isopentane. La consommation d'hydrogène est également suivie au cours du temps par la diminution de pression dans une bouteille réservoir située en amont du réacteur. L'activité catalytique est exprimée en moles de H₂ consommées par minute et par gramme de Ni.

Les activités catalytiques mesurées pour les catalyseurs A à G sont reportées dans le tableau 3 ci-après. Elles sont rapportées à l'activité catalytique (A_{HYD1}) mesurée pour le catalyseur A préparé dans les conditions classiques de réduction (à une température de 400°C pendant 16 heures sous flux d'hydrogène).

### Exemple 14 : Tests catalytiques : performances en hydrogénation du toluène (A_{HYD2})

Les catalyseurs A à G décrits dans les exemples ci-dessus sont également testés vis-à-vis de la réaction d'hydrogénation du toluène. La réaction d'hydrogénation sélective est opérée dans le même autoclave que celui décrit à l'exemple 13.

Dans un autoclave sont ajoutés 214 mL de n-heptane (fournisseur VWR^{®}, pureté > 99% chromanorm HPLC) et une quantité de 3 mL de catalyseur. L'autoclave est fermé et purgé. Ensuite l'autoclave est pressurisé sous 35 bar (3,5 MPa) d'hydrogène. Le catalyseur est d'abord réduit *in situ,* à 170 °C pendant 90 minutes sous un flux d'hydrogène de 1 L/h/g (rampe de montée en température de 1 °C/min) pour les catalyseurs A à G (ce qui correspond ici à l'étape g) du procédé de préparation selon l'invention selon un mode de réalisation). Après l'ajout de 216 mL de n-heptane (fournisseur VWR^{®}, pureté > 99% chromanorm HPLC), l'autoclave est fermé, purgé, puis pressurisé sous 35 bar (3,5 MPa) d'hydrogène, et porté à la température du test égale à 80°C. Au temps t=0, environ 26 g de toluène (fournisseur SDS^{®}, pureté > 99,8%) sont introduits dans l'autoclave (la composition initiale du mélange réactionnel est alors toluène 6 % pds / n-heptane 94 % pds) et l'agitation est mise en route à 1600 tr/min. La pression est maintenue constante à 35 bar (3,5 MPa) dans l'autoclave à l'aide d'une bouteille réservoir située en amont du réacteur. L'avancement de la réaction est suivi par prélèvement d'échantillons du milieu réactionnel à intervalles de temps réguliers : le toluène est totalement hydrogéné en méthylcyclohexane. La consommation d'hydrogène est également suivie au cours du temps par la diminution de pression dans une bouteille réservoir située en amont du réacteur. L'activité catalytique est exprimée en moles de H₂ consommées par minute et par gramme de Ni.

Les activités catalytiques mesurées pour les catalyseurs A à G sont reportées dans le tableau 3 ci-après. Elles sont rapportées à l'activité catalytique (A_{HYD2}) mesurée pour le catalyseur C . Pour comparaison, le catalyseur G a aussi été préparé dans les conditions classiques de réduction (à une température de 400°C pendant 16 heures sous flux d'hydrogène dans un réacteur en flux traversé en ex situ).

### Tableau 3

**Tableau 3 : Comparaison des performances en hydrogénation sélective d'un mélange contenant du styrène et de l'isoprène (A_{HYD1}) et en hydrogénation du toluène (A_{HYD2})**

| Catalyseur | Réduction (°C) | A_{HYD1} (%) | A_{HYD2} (%) |
|---|---|---|---|
| A (conforme) | 170°C,16h | 180 | 175 |
| B (conforme) | 170°C,16h | 175 | 170 |
| C (non conforme) | 170°C,16h | 100 | 100 |
| D (non conforme) | 170°C,16h | 60 | 70 |
| E (non conforme) | 170°C,16h | <1 | <1 |
| F(non conforme) | 170°C,16h | 55 | 45 |
| G (non conforme) | 170°C,16h | <1 | <1 |
| G (non conforme) | 400°C, 16h | 120 | 135 |

Ceci montre bien les performances en A_{HYD1} et A_{HYD2} améliorées des catalyseurs A, B selon l'invention, par rapport aux catalyseurs B à G non conformes. Le catalyseur A et B sont réduits à 170°C à hauteur de 90% et présente de petites tailles de particules réparties en « egg-shell ». Le catalyseur C présente de grosses particules du fait de l'utilisation de la solution S2 sans additifs. Son activité catalytique reste intéressante de fait de la présence de 80% de Ni réduit du fait de l'ajout de NiCu. Les catalyseurs D et F présentent bien de petites particules et réduites à hauteur de 90% mais elles ne sont pas réparties en croûte d'où l'activité en retrait. Les catalyseurs F et G malgré de petites particules ne sont pas actifs. L'absence de NiCu ne permet pas d'obtenir du Ni réduit, phase active en hydrogénation, à 170°C.

## Revendications

1. Catalyseur comprenant du nickel et du cuivre, à raison de 1 et 50 % en poids en élément nickel, mesurée par fluorescence X, par rapport au poids total du catalyseur, et d'un second élément métallique de cuivre, à raison de 0,5 à 15 % en poids en élément cuivre, mesurée par fluorescence X, par rapport au poids total du catalyseur, et un support d'alumine, ledit catalyseur étant **caractérisé en ce que** :
- le nickel est réparti à la fois sur une croûte en périphérie du support, et à coeur du support, l'épaisseur de ladite croûte, mesurée par microsonde de Castaing, étant comprise entre 2% et 15% du diamètre du catalyseur ;
- le ratio de densité en nickel entre la croûte et le coeur est supérieur strictement à 3 ;
- ladite croûte comprend plus de 25% en poids élément nickel par rapport au poids total de nickel contenu dans le catalyseur ;
- le ratio molaire entre le nickel et le cuivre est compris entre 0,5 et 5 ;
- au moins une partie du nickel et du cuivre se présente sous la forme d'un alliage de nickel-cuivre ;
- la teneur en nickel comprise dans l'alliage nickel-cuivre est comprise entre 0,5 et 15% en poids en élément nickel par rapport au poids total du catalyseur,
- la taille des particules de nickel, mesurée sous forme oxyde, dans le catalyseur est inférieure à 7 nm.

2. Catalyseur selon la revendication 1, dans lequel le ratio de densité en nickel entre la croûte et le coeur est supérieur ou égal à 3,5.

3. Catalyseur selon l'une des revendications 1 ou 2, dans lequel ladite croûte comprend plus de 40% en poids élément nickel par rapport au poids total de nickel contenu dans le catalyseur.

4. Catalyseur selon l'une quelconque des revendications 1 à 3, dans lequel l'intervalle de transition entre le coeur et la croûte du catalyseur est compris entre 0,05% et 3% du diamètre du catalyseur.

5. Catalyseur selon l'une quelconque des revendications 1 à 4, **caractérisé en ce que** la taille des particules de nickel dans le catalyseur est inférieure à 5 nm.

6. Catalyseur selon l'une quelconque des revendications 1 à 5, dans lequel la teneur en soufre du support d'alumine est comprise entre 0,001% et 2% poids par rapport au poids total du support d'alumine, et la teneur en sodium dudit support d'alumine est comprise entre 0,001% et 2% poids par rapport au poids total dudit gel d'alumine.

7. Catalyseur selon l'une quelconque des revendications 1 à 6, **caractérisé en ce que** l'épaisseur de ladite croûte est comprise entre 2,5% et 12% du diamètre du catalyseur.

8. Catalyseur selon l'une quelconque des revendications 1 à 7, **caractérisé en ce que** le ratio de densité en nickel entre la croûte et le coeur est compris entre 3,8 et 15.

9. Procédé de préparation d'un catalyseur selon l'une quelconque des revendications 1 à 8, ledit procédé étant **caractérisé en ce que** :
a) on approvisionne un gel ;
b) on met en forme le gel d'alumine de l'étape a) ;
c) on soumet le gel d'alumine mis en forme obtenu à l'issue de l'étape b) à un traitement thermique comprenant au moins une étape de traitement hydrothermal dans un autoclave en présence d'une solution acide, à une température comprise entre 100 et 800°C, et au moins une étape de calcination, à une température comprise entre 400 et 1500°C, réalisée après l'étape de traitement hydrothermal, pour obtenir un support d'alumine ;
d) on réalise l'enchaînement des sous-étapes suivantes :
d1) on met en contact le support d'alumine avec au moins un précurseur de nickel pour obtenir un précurseur de catalyseur,
d2) on sèche le précurseur de catalyseur obtenu à l'issue de l'étape d1) à une température inférieure à 250°C ;
d3) on met en contact le précurseur de catalyseur séché obtenu à l'issue de l'étape d2) avec au moins une solution contenant au moins un additif organique choisi parmi les aldéhydes renfermant 1 à 14 atomes de carbone par molécule, les cétones ou polycétones renfermant 3 à 18 atomes de carbone par molécule, les éthers et les esters renfermant 2 à 14 atomes de carbone par molécule, les alcools ou polyalcools renfermant 1 à 14 atomes de carbone par molécule et les acides carboxyliques ou polyacides carboxyliques renfermant 1 à 14 atomes de carbone par molécule, le ratio molaire entre l'additif organique et le nickel étant supérieur à 0,05 mol/mol ;
d4) on réalise un traitement hydrothermal du précurseur de catalyseur obtenu à l'issue de l'étape d3) à une température comprise entre 100 et 200°C pendant une durée comprise entre 30 minutes et 5 heures sous flux gazeux comprenant entre 5 et 650 grammes d'eau par kg de gaz sec ;
e) on réalise l'enchaînement des sous-étapes suivantes :
e1) on met en contact le support d'alumine avec au moins une solution contenant au moins un précurseur de cuivre et un précurseur de nickel à une concentration en nickel voulue pour obtenir sur le catalyseur final une teneur comprise entre 0,5 et 15 % poids en élément nickel par rapport au poids total du catalyseur final ;
e2) on réalise au moins une étape de séchage du précurseur de catalyseur obtenu à l'issue de l'étape e1) à une température inférieure à 250°C ;
les étapes d) et e) étant réalisées séparément dans un ordre indifférent,
f) on met en contact le support d'alumine avec au moins une solution contenant au moins un composé organique comprenant au moins une fonction acide carboxylique, ou au moins une fonction alcool, ou au moins une fonction ester, ou au moins une fonction amide, ou au moins une fonction amine,
l'étape f) étant réalisée, soit en même temps que la sous-étape d1) de l'étape d), soit avant soit après l'étape d), mais avant l'étape g), étant entendu que lorsque l'étape f) est réalisée avant ou après l'étape d), alors ladite étape f) inclut un séchage du précurseur de catalyseur à une température inférieure à 250°C après la mise en contact du support avec ladite solution comprenant au moins un composé organique;
g) on réduit le précurseur de catalyseur issu des étapes a) à f) par mise en contact dudit précurseur de catalyseur avec un gaz réducteur à une température supérieure ou égale à 150°C et inférieure à 250°C.

10. Procédé selon la revendication 9, dans lequel le rapport molaire entre ledit composé organique introduit à l'étape f) et l'élément nickel également introduit à l'étape d1) est compris entre 0,01 et 5,0 mol/mol.

11. Procédé selon l'une des revendications 9 ou 10, dans lequel les étapes d1) et f) sont réalisées simultanément.

12. Procédé selon l'une quelconque des revendications 9 à 11, dans lequel le composé organique de l'étape f) est choisi parmi l'acide oxalique, l'acide malonique, l'acide glycolique, l'acide acide lactique, l'acide tartronique, l'acide citrique, l'acide tartrique, l'acide pyruvique, l'acide lévulinique, l'éthylène glycol, le propane-1,3-diol, le butane-1,4-diol, le glycérol, le xylitol, le mannitol, le sorbitol, le diéthylène glycol, le glucose, la gamma valérolactone, le carbonate de diméthyle, le carbonate de diéthyle, la formamide, la N-méthylformamide, l'acétamide, la N-méthylacétamide, la N,N-diméthylméthanamide, la 2-pyrrolidone, la γ-lactame, la lactamide, l'urée, l'alanine, l'arginine, la lysine, la proline, la sérine, l'EDTA.

13. Procédé selon l'une quelconque des revendications 9 à 12, dans lequel le précurseur de cuivre est choisi parmi l'acétate de cuivre, l'acétylacétonate de cuivre, le nitrate de cuivre, le sulfate de cuivre, le chlorure de cuivre, le bromure de cuivre, l'iodure de cuivre ou le fluorure de cuivre.

14. Procédé selon l'une quelconque des revendications 9 à 13, dans lequel à l'étape d3) l'additif organique est choisi parmi l'acide formique, le formaldéhyde, l'acide acétique, l'acide citrique, l'acide oxalique, l'acide glycolique, l'acide malonique, l'éthanol, le méthanol, le formiate d'éthyle, le formiate de méthyle, le paraldéhyde, l'acétaldéhyde, l'acide gamma-valérolactone, le glucose, le sorbitol et le trioxane.

15. Procédé selon l'une quelconque des revendications 9 à 14, dans lequel le ratio molaire entre l'additif organique introduit à l'étape d2) et le nickel est compris entre 0,1 et 5 mol/mol.

16. Procédé selon l'une quelconque des revendications 9 à 15, dans lequel le composé organique de l'étape f) est différent de l'additif organique de l'étape d2).

17. Procédé d'hydrogénation sélective de composés polyinsaturés contenant au moins 2 atomes de carbone par molécule contenus dans une charge d'hydrocarbures ayant un point d'ébullition final inférieur ou égal à 300°C, lequel procédé étant réalisé à une température comprise entre 0 et 300°C, à une pression comprise entre 0,1 et 10 MPa, à un ratio molaire hydrogène/(composés polyinsaturés à hydrogéner) compris entre 0,1 et 10 et à une vitesse volumique horaire comprise entre 0,1 et 200 h⁻¹ lorsque le procédé est réalisé en phase liquide, ou à un ratio molaire hydrogène/(composés polyinsaturés à hydrogéner) compris entre 0,5 et 1000 et à une vitesse volumique horaire entre 100 et 40000 h⁻¹ lorsque le procédé est réalisé en phase gazeuse, en présence d'un catalyseur selon l'une quelconque des revendications 1 à 8.

18. Procédé d'hydrogénation d'au moins un composé aromatique ou polyaromatique contenu dans une charge d'hydrocarbures ayant un point d'ébullition final inférieur ou égal à 650°C, ledit procédé étant réalisé en phase gazeuse ou en phase liquide, à une température comprise entre 30 et 350°C, à une pression comprise entre 0,1 et 20 MPa, à un ratio molaire hydrogène/(composés aromatiques à hydrogéner) entre 0,1 et 10 et à une vitesse volumique horaire V.V.H. comprise entre 0,05 et 50 h⁻¹, en présence d'un catalyseur selon l'une quelconque des revendications 1 à 8.

## Patentansprüche

1. Katalysator, der Nickel und Kupfer mit einem Anteil von 1 und 50 Gew.-% eines Nickelelements, gemessen mittels Röntgenfluoreszenz, bezogen auf das Gesamtgewicht des Katalysators, und ein zweites metallisches Kupferelement mit einem Anteil von 0,5 bis 15 Gew.-% eines Kupferelements, gemessen mittels Röntgenfluoreszenz, bezogen auf das Gesamtgewicht des Katalysators, und einen Aluminiumoxid-Träger umfasst, wobei der Katalysator **dadurch gekennzeichnet, dass**:
- das Nickel sowohl auf einer Hülle am Umfang des Trägers als auch im Kern des Trägers verteilt ist, wobei die mit einer Castaing-Mikrosonde gemessene Dicke der Hülle zwischen 2 % und 15 % des Durchmessers des Katalysators beträgt;
- das Verhältnis der Nickeldichte zwischen der Hülle und dem Kern streng größer als 3 ist;
- die Hülle mehr als 25 Gew.-% des Nickelelements in Bezug auf das Gesamtgewicht des im Katalysator enthaltenen Nickels umfasst;
- das Stoffmengenverhältnis zwischen Nickel und Kupfer zwischen 0,5 und 5 liegt;
- zumindest ein Teil des Nickels und des Kupfers in Form einer Nickel-Kupfer-Legierung vorliegt;
- der Nickelgehalt in der Nickel-Kupfer-Legierung zwischen 0,5 und 15 Gew.-% des Nickelelements in Bezug auf das Gesamtgewicht des Katalysators liegt,
- die Größe der in Oxidform gemessenen Nickelpartikel im Katalysator kleiner als 7 nm ist.

2. Katalysator nach Anspruch 1, wobei das Verhältnis der Nickeldichte zwischen der Hülle und dem Kern größer als oder gleich 3,5 ist.

3. Katalysator nach einem der Ansprüche 1 oder 2, wobei die Hülle mehr als 40 Gew.-% des Nickelelements in Bezug auf das Gesamtgewicht des im Katalysator enthaltenen Nickels umfasst.

4. Katalysator nach einem der Ansprüche 1 bis 3, wobei das Übergangsintervall zwischen dem Kern und der Hülle des Katalysators zwischen 0,05 % und 3 % des Durchmessers des Katalysators liegt.

5. Katalysator nach einem der Ansprüche 1 bis 4, **dadurch gekennzeichnet, dass** die Größe der Nickelpartikel im Katalysator weniger als 5 nm beträgt.

6. Katalysator nach einem der Ansprüche 1 bis 5, wobei der Schwefelgehalt des Aluminiumoxid-Trägers zwischen 0,001 Gew.-% und 2 Gew.-%, bezogen auf das Gesamtgewicht des Aluminiumoxid-Trägers, liegt und der Natriumgehalt des Aluminiumoxid-Trägers zwischen 0,001 Gew.-% und 2 Gew.-%, bezogen auf das Gesamtgewicht des Aluminiumoxidgels, liegt.

7. Katalysator nach einem der Ansprüche 1 bis 6, **dadurch gekennzeichnet, dass** die Dicke der Hülle zwischen 2,5 % und 12 % des Durchmessers des Katalysators liegt.

8. Katalysator nach einem der Ansprüche 1 bis 7, **dadurch gekennzeichnet, dass** das Verhältnis der Dichte von Nickel zwischen der Hülle und dem Kern zwischen 3,8 und 15 liegt.

9. Verfahren zur Herstellung eines Katalysators nach einem der Ansprüche 1 bis 8, wobei das Verfahren **dadurch gekennzeichnet ist, dass**:
a) ein Gel bereitgestellt wird;
b) das Aluminiumoxidgel von Schritt a) geformt wird;
c) das nach Schritt b) erhaltene geformte Aluminiumoxidgel einer thermischen Behandlung, die mindestens einen Schritt einer hydrothermalen Behandlung in einem Autoklaven in Gegenwart einer sauren Lösung bei einer Temperatur zwischen 100 und 800 °C und mindestens einem Kalzinierungsschritt bei einer Temperatur zwischen 400 und 1500 °C umfasst, ausgesetzt wird, der nach dem Schritt der hydrothermalen Behandlung durchgeführt wird, wodurch ein Aluminiumoxid-Träger erhalten wird;
d) die Abfolge der folgenden Teilschritte durchgeführt wird:
d1) der Aluminiumoxid-Trägers wird mit mindestens einer Nickelvorstufe in Kontakt gebracht, wodurch eine Katalysatorvorstufe erhalten wird,
d2) die nach Schritt d1) erhaltene Katalysatorvorstufe wird bei einer Temperatur unter 250 °C getrocknet;
d3) die nach Schritt d2) erhaltene getrocknete Katalysatorvorstufe wird mit mindestens einer Lösung in Kontakt gebracht, die mindestens ein organisches Additiv enthält, das ausgewählt ist aus Aldehyden, die 1 bis 14 Kohlenstoffatome pro Molekül umfassen, Ketonen oder Polyketonen, die 3 bis 18 Kohlenstoffatome pro Molekül umfassen, Ethern und Estern, die 2 bis 14 Kohlenstoffatome pro Molekül umfassen, Alkoholen oder Polyalkoholen, die 1 bis 14 Kohlenstoffatome pro Molekül umfassen, und Carbonsäuren oder Polycarbonsäuren, die 1 bis 14 Kohlenstoffatome pro Molekül umfassen, wobei das Stoffmengenverhältnis zwischen dem organischen Additiv und dem Nickel mehr als 0,05 mol/mol beträgt;
d4) eine hydrothermale Behandlung der nach Schritt d3) erhaltenen Katalysatorvorstufe wird bei einer Temperatur zwischen 100 und 200 °C während einer Dauer zwischen 30 Minuten und 5 Stunden unter einem Gasstrom durchgeführt, der zwischen 5 und 650 g Wasser pro kg trockenem Gas umfasst;
e) die Abfolge der folgenden Teilschritte durchgeführt wird:
e1) der Aluminiumoxid-Träger wird mit mindestens einer Lösung in Kontakt gebracht, die mindestens eine Kupfervorstufe und eine Nickelvorstufe mit einer gewünschten Nickelkonzentration enthält, um auf dem endgültigen Katalysator einen Gehalt zwischen 0,5 und 15 Gew.-% des Nickelelements in Bezug auf das Gesamtgewicht des endgültigen Katalysators zu erhalten;
e2) mindestens ein Schritt des Trocknens der nach Schritt e1) erhaltenen Katalysatorvorstufe wird bei einer Temperatur unter 250 °C durchgeführt;
wobei die Schritte d) und e) getrennt in beliebiger Reihenfolge durchgeführt werden,
f) der Aluminiumoxid-Träger mit mindestens einer Lösung in Kontakt gebracht wird, die mindestens eine organische Verbindung enthält, die mindestens eine Carbonsäurefunktion oder mindestens eine Alkoholfunktion oder mindestens eine Esterfunktion oder mindestens eine Amidfunktion oder mindestens eine Aminfunktion umfasst,
wobei Schritt f) entweder gleichzeitig mit dem Teilschritt d1) von Schritt d) oder vor oder nach Schritt d), aber vor Schritt g) durchgeführt wird, wobei als vereinbart gilt, dass, wenn Schritt f) vor oder nach Schritt d) durchgeführt wird, Schritt f) dann ein Trocknen der Katalysatorvorstufe bei einer Temperatur unter 250 °C nach dem In-Kontakt-Bringen des Trägers mit der mindestens eine organische Verbindung umfassenden Lösung einschließt;
g) die Katalysatorvorstufe aus den Schritten a) bis f) reduziert wird, indem die Katalysatorvorstufe mit einem reduzierenden Gas bei einer Temperatur über oder gleich 150 °C und unter 250 °C in Kontakt gebracht wird.

10. Verfahren nach Anspruch 9, wobei das Stoffmengenverhältnis zwischen der in Schritt f) eingeführten organischen Verbindung und dem in Schritt d1) ebenfalls eingeführten Nickelelement zwischen 0,01 und 5,0 mol/mol liegt.

11. Verfahren nach einem der Ansprüche 9 oder 10, wobei die Schritte d1) und f) gleichzeitig durchgeführt werden.

12. Verfahren nach einem der Ansprüche 9 bis 11, wobei die organische Verbindung von Schritt f) ausgewählt ist aus Oxalsäure, Malonsäure, Glycolsäure, Milchsäure, Tartronsäure, Citronensäure, Weinsäure, Brenztraubensäure, Lävulinsäure, Ethylenglycol, Propan-1,3-diol, Butan-1,4-diol, Glycerin, Xylit, Mannit, Sorbit, Diethylenglycol, Glucose, Gamma-Valerolacton, Dimethylcarbonat, Diethylcarbonat, Formamid, N-Methylformamid, Acetamid, N-Methylacetamid, N,N-Dimethylmethanamid, 2-Pyrrolidon, γ-Lactam, Lactamid, Harnstoff, Alanin, Arginin, Lysin, Prolin, Serin, EDTA.

13. Verfahren nach einem der Ansprüche 9 bis 12, wobei die Kupfervorstufe ausgewählt ist aus Kupferacetat, Kupferacetylacetonat, Kupfernitrat, Kupfersulfat, Kupferchlorid, Kupferbromid, Kupferjodid oder Kupferfluorid.

14. Verfahren nach einem der Ansprüche 9 bis 13, wobei in Schritt d3) das organische Additiv aus Ameisensäure, Formaldehyd, Essigsäure, Citronensäure, Oxalsäure, Glycolsäure, Malonsäure, Ethanol, Methanol, Ethylformiat, Methylformiat, Paraldehyd, Acetaldehyd, Gamma-Valerolactonsäure, Glucose, Sorbit und Trioxan ausgewählt ist.

15. Verfahren nach einem der Ansprüche 9 bis 14, wobei das Stoffmengenverhältnis zwischen dem in Schritt d2) eingeführten organischen Additiv und dem Nickel zwischen 0,1 und 5 mol/mol liegt.

16. Verfahren nach einem der Ansprüche 9 bis 15, wobei die organische Verbindung von Schritt f) vom organischen Additiv von Schritt d2) verschieden ist.

17. Verfahren zur selektiven Hydrierung von mehrfach ungesättigten Verbindungen, die mindestens 2 Kohlenstoffatome pro Molekül enthalten, die in einem Kohlenwasserstoff-Einsatzmaterial mit einem Endsiedepunkt unter oder gleich 300 °C enthalten sind, wobei das Verfahren bei einer Temperatur zwischen 0 und 300 °C, bei einem Druck zwischen 0,1 und 10 MPa, bei einem Stoffmengenverhältnis Wasserstoff/(zu hydrierende mehrfach ungesättigte Verbindungen) zwischen 0,1 und 10 und bei einer stündlichen Raumgeschwindigkeit zwischen 0,1 und 200 h⁻¹, wenn das Verfahren in der Flüssigphase durchgeführt wird, oder bei einem Stoffmengenverhältnis Wasserstoff/(zu hydrierende mehrfach ungesättigte Verbindungen) zwischen 0,5 und 1000 und bei einer stündlichen Raumgeschwindigkeit zwischen 100 und 40 000 h⁻¹, wenn das Verfahren in der Gasphase durchgeführt wird, in Gegenwart eines Katalysators nach einem der Ansprüche 1 bis 8 durchgeführt wird.

18. Verfahren zur Hydrierung mindestens einer aromatischen oder polyaromatischen Verbindung, die in einem Kohlenwasserstoff-Einsatzmaterial mit einem Endsiedepunkt unter oder gleich 650 °C enthalten ist, wobei das Verfahren in der Gasphase oder in der Flüssigphase, bei einer Temperatur zwischen 30 und 350 °C, bei einem Druck zwischen 0,1 und 20 MPa, einem Stoffmengenverhältnis Wasserstoff/(zu hydrierende mehrfach ungesättigte Verbindungen) zwischen 0,1 und 10 und bei einer stündlichen Raumgeschwindigkeit, V.V.H., zwischen 0,05 und 50 h⁻¹ in Gegenwart eines Katalysators nach einem der Ansprüche 1 bis 8 durchgeführt wird.

## Claims

1. Catalyst comprising nickel and copper, in a proportion of 1% and 50% by weight of nickel element, measured by x-ray fluorescence, relative to the total weight of the catalyst, and a second metallic element of copper, in a proportion of 0.5% to 15% by weight of copper element, measured by x-ray fluorescence, relative to the total weight of the catalyst, and an alumina support, said catalyst being **characterized in that**:
- the nickel is distributed both on a crust at the periphery of the support, and in the core of the support, the thickness of said crust, measured by Castaing microprobe, being between 2% and 15% of the diameter of the catalyst;
- the nickel density ratio between the crust and the core is strictly greater than 3;
- said crust comprises more than 25% by weight of nickel element relative to the total weight of nickel contained in the catalyst;
- the mole ratio between nickel and copper is between 0.5 and 5;
- at least one portion of the nickel and copper is in the form of a nickel-copper alloy;
- the nickel content in the nickel-copper alloy is between 0.5% and 15% by weight of nickel element relative to the total weight of the catalyst,
- the size of the nickel particles, measured in oxide form, in the catalyst is less than 7 nm.

2. Catalyst according to Claim 1, wherein the nickel density ratio between the crust and the core is greater than or equal to 3.5.

3. Catalyst according to either of Claims 1 and 2, wherein said crust comprises more than 40% by weight of nickel element relative to the total weight of nickel contained in the catalyst.

4. Catalyst according to any one of Claims 1 to 3, wherein the transition interval between the core and the crust of the catalyst is between 0.05% and 3% of the diameter of the catalyst.

5. Catalyst according to any one of Claims 1 to 4, **characterized in that** the size of the nickel particles in the catalyst is less than 5 nm.

6. Catalyst according to any one of Claims 1 to 5, wherein the sulfur content of the alumina support is between 0.001% and 2% by weight relative to the total weight of the alumina support, and the sodium content of said alumina support is between 0.001% and 2% by weight relative to the total weight of said alumina gel.

7. Catalyst according to any one of Claims 1 to 6, **characterized in that** the thickness of said crust is between 2.5% and 12% of the diameter of the catalyst.

8. Catalyst according to any one of Claims 1 to 7, **characterized in that** the nickel density ratio between the crust and the core is between 3.8 and 15.

9. Process for preparing a catalyst according to any one of Claims 1 to 8, said process being **characterized in that**:
a) a gel is provided;
b) the alumina gel from step a) is shaped;
c) the shaped alumina gel obtained at the end of step
b) is subjected to a heat treatment comprising at least one hydrothermal treatment step in an autoclave in the presence of an acid solution, at a temperature of between 100°C and 800°C, and at least one calcining step, at a temperature of between 400°C and 1500°C, carried out after the hydrothermal treatment step, in order to obtain an alumina support;
d) the sequence of the following sub-steps is carried out:
d1) the alumina support is brought into contact with at least one nickel precursor in order to obtain a catalyst precursor,
d2) the catalyst precursor obtained at the end of step d1) is dried at a temperature below 250°C;
d3) the dried catalyst precursor obtained at the end of step d2) is brought into contact with at least one solution containing at least one organic additive chosen from aldehydes containing 1 to 14 carbon atoms per molecule, ketones or polyketones containing 3 to 18 carbon atoms per molecule, ethers and esters containing 2 to 14 carbon atoms per molecule, alcohols or polyalcohols containing 1 to 14 carbon atoms per molecule and carboxylic acids or polycarboxylic acids containing 1 to 14 carbon atoms per molecule, the mole ratio between the organic additive and the nickel being greater than 0.05 mol/mol;
d4) a hydrothermal treatment of the catalyst precursor obtained at the end of step d3) is carried out at a temperature between 100°C and 200°C for a period of between 30 minutes and 5 hours under a gas stream comprising between 5 and 650 grams of water per kg of dry gas;
e) the sequence of the following sub-steps is carried out:
e1) the alumina support is brought into contact with at least one solution containing at least one copper precursor and one nickel precursor at a desired nickel concentration in order to obtain, on the final catalyst, a content of between 0.5% and 15% by weight of nickel element relative to the total weight of the final catalyst;
e2) at least one step of drying the catalyst precursor obtained at the end of step e1) is carried out at a temperature below 250°C;
steps d) and e) being carried out separately in any order,
f) the alumina support is brought into contact with at least one solution containing at least one organic compound comprising at least one carboxylic acid function, or at least one alcohol function, or at least one ester function, or at least one amide function, or at least one amine function,
step f) being carried out, either at the same time as sub-step d1) of step d), or before or after step d), but before step g), it being understood that when step f) is carried out before or after step d), then said step f) includes drying of the catalyst precursor at a temperature below 250°C after bringing the support into contact with said solution comprising at least one organic compound;
g) the catalyst precursor resulting from steps a) to f) is reduced by bringing said catalyst precursor into contact with a reducing gas at a temperature above or equal to 150°C and below 250°C.

10. Process according to Claim 9, wherein the mole ratio between said organic compound introduced in step f) and the nickel element also introduced in step d1) is between 0.01 and 5.0 mol/mol.

11. Process according to either of Claims 9 and 10, wherein steps d1) and f) are carried out at the same time.

12. Process according to any one of Claims 9 to 11, wherein the organic compound of step f) is chosen from oxalic acid, malonic acid, glycolic acid, lactic acid, tartronic acid, citric acid, tartaric acid, pyruvic acid, levulinic acid, ethylene glycol, propane-1,3-diol, butane-1,4-diol, glycerol, xylitol, mannitol, sorbitol, diethylene glycol, glucose, gamma-valerolactone, dimethyl carbonate, diethyl carbonate, formamide, N-methylformamide, acetamide, N-methylacetamide, N,N-dimethylmethanamide, 2-pyrrolidone, γ-lactam, lactamide, urea, alanine, arginine, lysine, proline, serine, EDTA.

13. Process according to any one of Claims 9 to 12, wherein the copper precursor is chosen from copper acetate, copper acetylacetonate, copper nitrate, copper sulfate, copper chloride, copper bromide, copper iodide or copper fluoride.

14. Process according to any one of Claims 9 to 13, wherein, in step d3), the organic additive is chosen from formic acid, formaldehyde, acetic acid, citric acid, oxalic acid, glycolic acid, malonic acid, ethanol, methanol, ethyl formate, methyl formate, paraldehyde, acetaldehyde, gamma-valerolactone acid, glucose, sorbitol and trioxane.

15. Process according to any one of Claims 9 to 14, wherein the mole ratio between the organic additive introduced in step d2) and the nickel is between 0.1 and 5 mol/mol.

16. Process according to any one of Claims 9 to 15, wherein the organic compound of step f) is different from the organic additive of step d2).

17. Process for the selective hydrogenation of polyunsaturated compounds containing at least 2 carbon atoms per molecule, contained in a hydrocarbon feedstock having a final boiling point below or equal to 300°C, said process being carried out at a temperature of between 0°C and 300°C, at a pressure of between 0.1 and 10 MPa, at a hydrogen/(polyunsaturated compounds to be hydrogenated) mole ratio of between 0.1 and 10 and at an hourly space velocity of between 0.1 and 200 h⁻¹ when the process is carried out in the liquid phase, or at a hydrogen/(polyunsaturated compounds to be hydrogenated) mole ratio of between 0.5 and 1000 and at an hourly space velocity of between 100 and 40 000 h⁻¹ when the process is carried out in the gas phase, in the presence of a catalyst according to any one of Claims 1 to 8.

18. Process for the hydrogenation of at least one aromatic or polyaromatic compound contained in a hydrocarbon feedstock having a final boiling point below or equal to 650°C, said process being carried out in the gas phase or in the liquid phase, at a temperature of between 30°C and 350°C, at a pressure of between 0.1 and 20 MPa, at a hydrogen/(aromatic compounds to be hydrogenated) mole ratio of between 0.1 and 10 and at an hourly space velocity HSV of between 0.05 and 50 h⁻¹, in the presence of a catalyst according to any one of Claims 1 to 8.
